# EUROPÄISCHE PATENTANMELDUNG

(11) **EP 4 082 581 A1**
(43) Veröffentlichungstag der Anmeldung: **02.11.2022**
(21) Anmeldenummer: 22174349.5
(22) Anmeldetag: 21.10.2019
(51) Int. Cl.: A61K 51/04, A61K 101/02, A61K 103/00, A61K 103/10, A61K 103/30, A61K 103/40, C07C 275/16, C07C 323/60, C07D 245/02, C07D 253/02, C07D 257/02, C07D 401/14, C07D 403/12, C07F 5/02, C07F 9/38, C07F 9/6506, C07F 9/6515, C07F 9/6558

(54) **MARKIERUNGSVORLÄUFER MIT QUADRATSÄURE-KOPPLUNG**

(30) Priorität: 24.10.2018 DE 102018126558
(62) Teilanmeldung aus: 19800930.0
(71) Anmelder: SCV - SpezialChemikalienVertrieb GmbH, 10243 Berlin (DE)
(72) Erfinder: Rösch, Frank, 10243 Berlin (DE); Grafenstein, Lukas, 55116 Mainz (DE); Engelbogen, Niels, 24118 Kiel (DE); Bergmann, Ralf, 01328 Dresden (DE)
(74) Vertreter: Zounek, Alexis Nikolai

(57) **Zusammenfassung**

Ein Markierungsvorläufer umfasst einen Chelator für die Radiomarkierung mit ⁴⁴Sc, ⁴⁷Sc, ⁵⁵Co, ⁶²Cu , ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰M, ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi und ²²⁵Ac und zwei biologische Targetingvektoren, die über Quadratsäuregruppen mit dem Chelator gekoppelt sind.

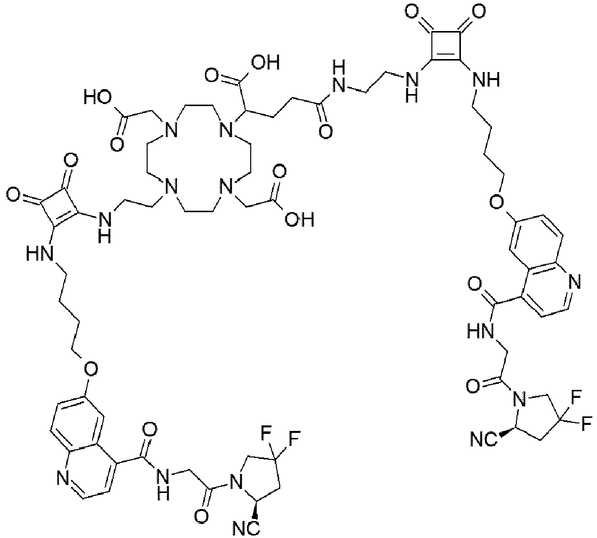

## Beschreibung

Die vorliegende Erfindung betrifft einen Markierungsvorläufer, umfassend einen Chelator und zwei, jeweils über Linker und Spacer mit dem Chelator gekoppelte Targetingvektoren.

Der erfindungsgemäße Markierungsvorläufer ist vorgesehen für die bildgebende nuklearmedizinische, radiologische Diagnose und Behandlung (Theranostik) von Krebserkrankungen, bei denen Tumorzellen Fibroblasten-Aktivierungs-Protein (FAP) exprimieren.

In der klinischen Behandlung werden seit etwa 15 Jahren in zunehmendem Umfang bildgebende radiologische Diagnoseverfahren, wie Positronen-Emissions-Tomographie (PET) und Einzelphotonen-Emissionscomputertomographie (single photon emission computed tomography, SPECT) eingesetzt. Jüngst gewinnen auch theranostische Verfahren zunehmend an Bedeutung.

In der radiologischen Diagnostik und Theranostik werden Tumorzellen mit einem radioaktiven Isotop, wie beispielsweise ⁶⁸Ga oder ¹⁷⁷Lu markiert bzw. bestrahlt. Hierbei werden Markierungsvorläufer eingesetzt, die das jeweilige Radioisotop kovalent (¹⁸F) oder koordinativ (⁶⁸Ga, ^{99m}Tc, ¹⁷⁷Lu) binden. Die Markierungsvorläufer umfassen im Fall von metallischen Radioisotopen als wesentliche chemische Komponente einen Chelator für die effektive und stabile Komplexierung des Radioisotops sowie als funktionelle Komponente einen biologischen Targetingvektor, der an Zielstrukturen im Tumorgewebe bindet. In der Regel weist der biologische Targetingvektor eine hohe Affinität zu zellmembranständigen Rezeptoren, Proteinen oder Enzymen von Tumorzellen auf.

Nach intravenöser Injektion in den Blutkreislauf reichert sich der mit einem Radioisotop markierte Markierungsvorläufer auf bzw. in Tumorzellen an. Um die Strahlendosis bei diagnostischen Untersuchungen im gesundem Gewebe zu minimieren, wird eine geringe Menge eines Radioisotops mit kurzer Halbwertszeit von wenigen Stunden bis Tagen verwendet.

Durch den Chelator oder die Fluorierungsgruppe werden die Konfiguration und chemischen Eigenschaften des Targetingvektors modifiziert und in der Regel dessen Affinität zu Tumorzellen stark beeinflusst. Dementsprechend wird die Kopplung zwischen dem Chelator oder der Fluorierungsgruppe und dem mindestens einen Targetingvektor in aufwendigen Trialand-Error Versuchen bzw. sogenannten biochemischen Screenings maßgeschneidert. Hierbei wird eine große Zahl von, den Chelator oder eine Fluorierungsgruppe und den mindestens einen Targetingvektor umfassenden Markierungsvorläufern synthetisiert und insbesondere die Affinität zu Tumorzellen quantifiziert. Der Chelator oder die Fluorierungsgruppe und die chemische Kopplung mit dem Targetingvektor sind maßgeblich für die biologische und radiologische Potenz des jeweiligen Markierungsvorläufers.

Zusätzlich zu einer hohen Affinität muss der Markierungsvorläufer weitere Anforderungen erfüllen, wie
- schnelle und effektive Komplexierung oder kovalente Bindung des jeweiligen Radioisotops;
- hohe Selektivität für Tumorzellen relativ zu gesundem Gewebe;
- in vivo Stabilität, d. h. biochemische Beständigkeit in Blutserum unter physiologischen Bedingungen.

### Prostatakrebs

Für Männer in den Industrieländern ist Prostatakrebs die häufigste Krebsart und die dritthäufigste tödliche Krebserkrankung. Das Tumorwachstum schreitet bei dieser Erkrankung nur langsam voran und bei einer Diagnose im frühen Stadium liegt die 5-Jahre Überlebensrate bei nahezu 100%. Wird die Krankheit erst entdeckt, wenn der Tumor metastasiert hat, sinkt die Überlebensrate dramatisch. Ein zu frühes und zu aggressives Vorgehen gegen den Tumor kann wiederum die Lebensqualität des Patienten unnötig beeinträchtigen. So kann z. B. die operative Entfernung der Prostata zu Inkontinenz und Impotenz führen. Eine sichere Diagnose und Informationen über das Stadium der Krankheit sind essentiell für eine erfolgreiche Behandlung mit hoher Lebensqualität des Patienten. Ein weitverbreitetes Diagnosemittel neben dem Abtasten der Prostata durch einen Arzt ist die Bestimmung von Tumormarkern im Blut des Patienten. Der prominenteste Marker für ein Prostatakarzinom ist die Konzentration des prostataspezifischen Antigens (PSA) im Blut. Allerdings ist die Aussagekraft der PSA-Konzentration umstritten, da Patienten mit leicht erhöhten Werten oft kein Prostatakarzinom haben, jedoch 15% der Patienten mit Prostatakarzinom keine erhöhte PSA-Konzentration im Blut zeigen. Eine weitere Zielstruktur für die Diagnose von Prostatatumoren ist das prostataspezifische Membranantigen (PSMA). Im Gegensatz zu PSA kann PSMA im Blut nicht nachgewiesen werden. Es ist ein membrangebundenes Glykoprotein mit enzymatischer Aktivität. Seine Aufgabe ist die Abspaltung von C-terminalem Glutamat von N-Acetyl-Aspartyl-Glutamat (NAAG) und Folsäure-(poly)-γ-Glutamat. PSMA tritt in normalem Gewebe kaum auf, wird aber von Prostatakarzinomzellen stark überexprimiert, wobei die Expression mit dem Stadium der Tumorerkrankung eng korreliert. Auch Lymphknoten- und Knochenmetastasen von Prostatakarzinomen zeigen zu 40% eine Expression von PSMA.

Eine Strategie des molekularen Targetings von PSMA besteht darin, mit Antikörpern an die Proteinstruktur des PSMA zu binden. Eine weitere Herangehensweise ist die enzymatische Aktivität von PSMA, die gut verstanden ist, zu nutzen. In der enzymatischen Bindungstasche von PSMA befinden sich zwei Zn²⁺-Ionen, die Glutamat binden. Vor dem Zentrum mit den beiden Zn²⁺-Ionen befindet sich eine aromatische Bindungstasche. Das Protein ist in der Lage sich aufzuweiten und an die Bindungspartner anzupassen (induced fit), so dass es neben NAAG auch Folsäure binden kann, wobei die Pteroinsäuregruppe in der aromatischen Bindungstasche andockt. Die Nutzung der enzymatischen Affinität von PSMA ermöglicht die Aufnahme des Substrates in die Zelle (Endozytose) unabhängig von einer enzymatischen Spaltung des Substrates.

Daher eignen sich insbesondere PSMA-Inhibitoren gut als Targetingvektoren für bildgebende diagnostische und theranostische Radiopharmazeutika bzw. Radiotracer. Die radioaktiv markierten Inhibitoren binden an das aktive Zentrum des Enzyms, werden dort jedoch nicht umgesetzt. Die Bindung zwischen dem Inhibitor und dem radioaktiven Label wird also nicht gelöst. Begünstigt durch Endozytose wird der Inhibitor mit dem radioaktiven Label in die Zelle aufgenommen und in den Tumorzellen angereichert.

Inhibitoren mit hoher Affinität zu PSMA enthalten in der Regel ein Glutamat-Motiv und eine enzymatisch nicht spaltbare Struktur. Ein hoch effektiver PSMA-Inhibitor ist 2-Phosphonomethyl-Glutarsäure bzw. 2-Phosphonomethyl-Pentandisäure (2-PMPA), in der das Glutamat-Motiv an eine durch PSMA nicht spaltbare Phosphonatgruppe gebunden ist. Eine weitere Gruppe von PSMA-Inhibitoren, die in den klinisch relevanten Radiopharmaka PSMA-11 und PSMA-617 genutzt wird, bilden Harnstoff-basierte Inhibitoren.

Es hat sich als vorteilhaft erwiesen, zusätzlich zu der Bindungstasche für das Glutamat-Motiv die aromatische Bindungstasche von PSMA zu adressieren. Beispielsweise ist in dem hoch wirksamen Radiopharmakon PSMA-11 das Bindungsmotiv L-Lysin-Urea-L-Glutamat (KuE) über Hexyl (Hexyl-Linker) an einen aromatischen HBED-Chelator (N,N'-Bis(2-hydroxy-5-(ethylen-beta-carboxy)benzyl)ethylendiamin N,N'-diacetat) gebunden.

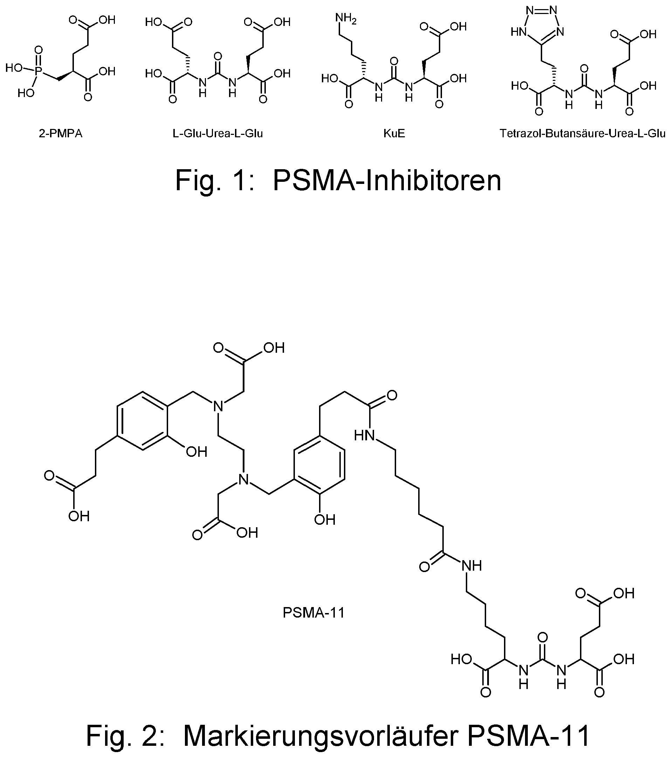

Wird L-Lysin-Urea-L-Glutamat (KuE) hingegen an den nicht-aromatischen Chelator DOTA (1,4,7,10-Tetraazacyclododecan-1,4,7,10-tetraacetat) gebunden, so ist eine verminderte Affinität und Anreicherung in Tumorgewebe zu konstatieren. Um dennoch den DOTA-Chelator für ein PSMA-affines Radiopharmakon mit therapeutischen Radioisotopen, wie ¹⁷⁷Lu oder ²²⁵Ac nutzen zu können, muss der Linker angepasst werden. Mittels gezielter Substitution von Hexyl durch verschiedene aromatische Strukturen wurde das hoch wirksame Radiopharmakon PSMA-617, der derzeitige Goldstandard, gefunden (Fig. 1: PSMA-Inhibitoren; Fig. 2: Markierungsvorläufer PSMA-11; Fig. 3: Markierungsvorläufer PSMA-617).

### Tumorstroma

Viele Tumore umfassen maligne Epithelzellen und sind von mehreren nicht-kanzerogenen Zellpopulationen umgeben, einschließlich aktivierten Fibroblasten, Endothelzellen, Perizyten, Immunregulationszellen und Zytokinen in der extrazellulären Matrix. Diese sogenannten Stromazellen, die den Tumor umgeben, spielen eine wichtige Rolle bei der Entwicklung, dem Wachstum und der Metastasierung von Karzinomen. Ein großer Teil der Stromazellen sind aktivierte Fibroblasten, die als krebsassoziierte Fibroblasten (CAFs) bezeichnet werden. Im Laufe der Tumorprogression verändern CAFs ihre Morphologie und biologische Funktion. Diese Veränderungen werden durch interzelluläre Kommunikation zwischen Krebszellen und CAFs induziert. Hierbei bilden CAFs eine Mikroumgebung, die das Wachstum der Krebszellen begünstigt. Es hat sich gezeigt, dass allein auf Krebszellen zielende Therapien unzulänglich sind. Effektive Therapien müssen die Tumor-Mikroumgebung, d. h. CAFs einbeziehen. Bei mehr als 90 % aller menschlichen Karzinome wird von CAFs das Fibroblasten-Aktivierungs-Protein (FAP) überexprimiert. Daher repräsentiert FAP einen erfolgversprechenen Angriffspunkt für die radiologische Diagnostik und Theranostik. Als affine biologische Targetingvektoren für FAP-Markierungsvorläufer eignen sich - analog zu PSMA - insbesondere FAP-Inhibitoren (FAPI oder FAPi). FAP weist bimodale, durch dasselbe aktive Zentrum katalysierte Aktivität von Dipeptidylpeptidasen (DPP) und Prolyloligopeptidasen (PREP) auf. Dementsprechend kommen zwei Arten von Inhibitoren in Betracht, welche die DPP- und/oder die PREP-Aktivität von FAP hemmen. Bekannte Inhibitoren für die PREP-Aktivität von FAP weisen eine niedrige Selektivität für FAP auf. Bei Krebsarten, bei denen sowohl FAP als auch PREP überexprimiert wird, können jedoch auch PREP-Inhibitoren trotz ihrer geringen FAP-Selektivität als Targetingvektoren geeignet sein.

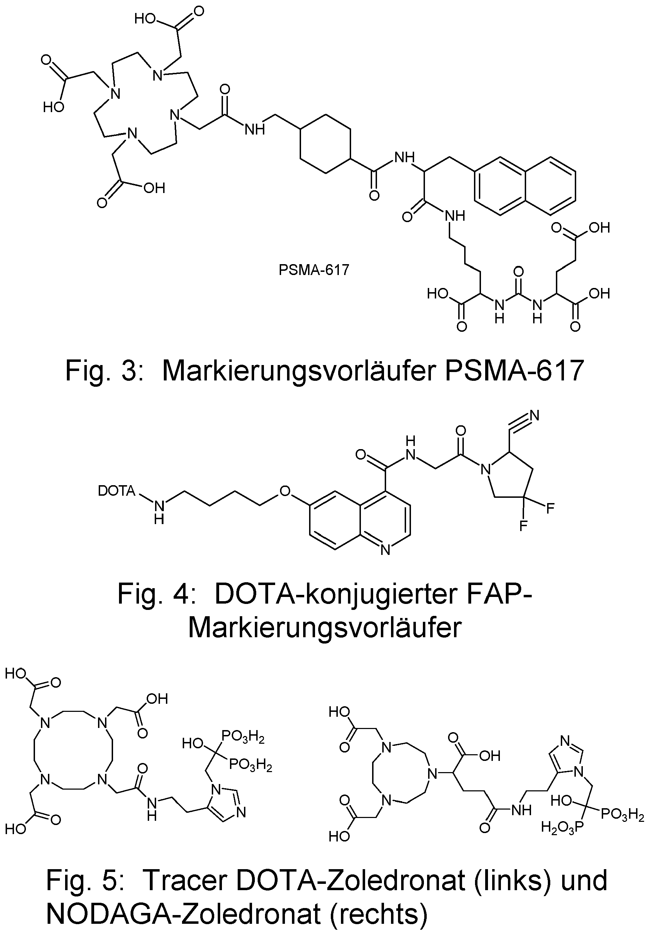

Fig. 4 zeigt einen DOTA-konjugierten FAP-Markierungsvorläufer, bei dem der Chelator an die pharmakophore Einheit ((S)-N-(2-(2-Cyano-4,4-difluoropyrolidin-1-yl)-2-oxoethyl)-6-(4-aminobutyloxy)-quinolin-4-carboxamid über die 4-aminobutoxy-Funktionalität an das Quinolin gekoppelt ist (Fig. 4: DOTA-konjugierter FAP-Markierungsvorläufer).

### Knochenmetastasen

Knochenmetastasen exprimieren Farnesyl-Pyrophosphat-Synthase (FPPS), ein Enzym im HMG-CoA-Reduktase-(Mevalonat)-Weg. Durch die Hemmung von FPPS wird die Produktion von Farnesyl, einem wichtigen Molekül für das Docking von Signalproteinen an der Zellmembran unterdrückt. Als Folge wird die Apoptose von kanzerogenen Knochenzellen induziert. FPPS wird durch Bisphosphonate, wie Alendronat, Pamidronat und Zoledronat inhibiert. Beispielweise wird der Tracer BPAMD mit dem Targetingvektor Pamindronat regelmäßig bei der Behandlung von Knochenmetastasen eingesetzt.

Als besonders effektiver Tracer für die Theranostik von Knochenmetastasen hat sich Zoledronat (ZOL), ein Hydroxy-Bisphosphonat mit einer heteroaromatischen N-Einheit erwiesen. Mit den Chelatoren NODAGA- und DOTA-konjugiertes Zolendronat (Fig. 5) sind die derzeit potentesten Radio-Theranostika für Knochenmetastasen (Fig. 5: Tracer DOTA-Zoledronat (links) und NODAGA-Zoledronat (rechts)).

Im Stand der Technik ist eine Vielzahl von Markierungsvorläufern für Diagnose und Theranostik von Krebserkrankungen mit radioaktiven Isotopen bekannt.

WO 2015055318 A1 offenbart Radiotracer für die Diagnose und Theranostik von Prostata- oder epithelialen Karzinomen, wie unter anderem, die in Fig. 3 gezeigte Verbindung PSMA-617.

Die vorliegende Erfindung hat die Aufgabe, für Diagnose und Theranostik von Prostata- und Stromakarzinomen einen effizienten Markierungsvorläufer für Radiotracer mit hoher Tumorselektivität und -dosis bereitzustellen.

Diese Aufgabe wird gelöst durch einen Markierungsvorläufer mit der Struktur 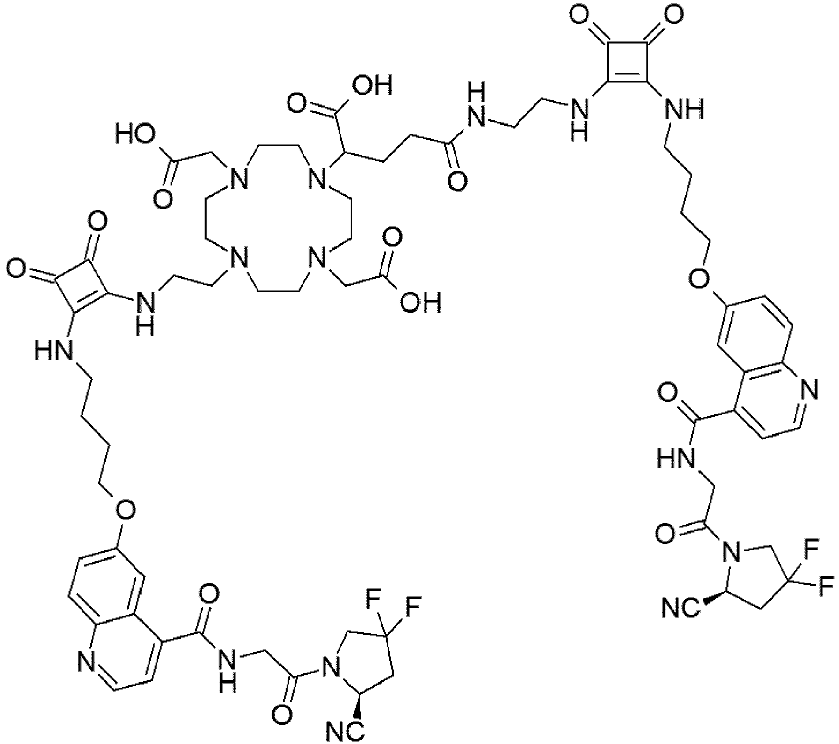 Andere nicht anspruchsgemäße Markierungsvorläufer haben die Struktur (A), (B), (C), (D), (E), (F), (G), (H), (I), (J), (K) oder (L) mit
(A) = **Ch**-L₁-QS-TV₁ ,
(B) = **Ch**-L₁-QS-S₁-TV₁ ,
(C) = **Ch**-L₁-QS-S₁-QS-TV₁ ,
(D) = **Ch**-L₁-QS-Sz-QS-S₁-TV₁ ,
(E) = TV₂-QS-L₂-**Ch**-L₁-QS-TV₁ ,
(F) = TV₂-S₃-QS-L₂-**Ch**-L₁-QS-S₁-TV₁ ,
(G) = TV₂-QS-S₄-QS-L₂-**Ch**-L₁-QS-S₂-QS-TV₁ ,
(H) = TV₂-S₃-QS-S₄-QS-L₂-**Ch**-L₁-QS-S₂-QS-S₁-TV₁ ,
(I) = **Fg**-L₁-QS-TV₁ ,
(J) = **Fg**-L₁-QS-S₁-TV₁ ,
(K) = **Fg**-L₁-QS-S₂-QS-TV₁ ,
(L) = **Fg**-L₁-QS-S₂-QS-S₁-TV₁ ;
umfassend
- einen Chelator Ch, gewählt aus der Gruppe, umfassend EDTA (Ethylendiamintetraacetat), EDTMP (Diethylentriaminpenta(methylenphosphonsäure)), DTPA (Diethylentriaminpentaacetat) und dessen Derivate, DOTA (Dodeca-1,4,7,10-tetraamin-tetraacetat), DOTAGA (2-(1,4,7,10-Tetraazacyclododecan-4,7,10)-pentandisäure) und anderen DOTA-Derivaten, TRITA (Trideca-1,4,7,10-tetraamin-tetraacetat), TETA (Tetradeca-1,4,8,11-tetraamin-tetra-acetat) und dessen Derivate, NOTA (Nona-1,4,7-triamin-triacetat) und dessen Derivate wie beispielsweise NOTAGA (1,4,7-triazacyclononan,1-glutarsäure,4,7-acetat), NOPO (1,4,7-triazacyclononan-1,4-bis[methylen(hydroxymethyl)phosphinsäure]-7-[methylen(2-carboxyethyl)phosphinsäure]), PEPA (Pentadeca-1,4,7,10,13-pentaaminpentaacetat), HEHA (Hexadeca-1,4,7,10,13,16-hexaamin-hexaacetat) und dessen Derivate, HBED (Hydroxybenzyl-ethylen-diamin) und dessen Derivate, DEDPA und dessen Derivate, wie H₂DEDPA (1,2-[[6-(carboxylat-)pyridin-2-yl]methylamin]ethan), DFO (Deferoxamin) und dessen Derivate, Trishydroxypyridinon (THP) und dessen Derivate wie YM103, TRAP (Triazacyclononan-phosphinsäure), TEAP (Tetraazycyclodecan-phosphinsäure) und dessen Derivate, AAZTA (6-Amino-6-methylperhydro-1,4-diazepin-N,N,N',N'-tetraacetat) und Derivate wie DATA ((6-Pentansäure)-6-(amino)methyl-1,4-diazepin triacetat); SarAr (1-N-(4-aminobenzyl)-3,6,10,13,16,19-hexaazabicyclo[6.6.6]-eicosan-1,8-diamin) und Salze davon, Aminothiole und deren Derivate des Typs 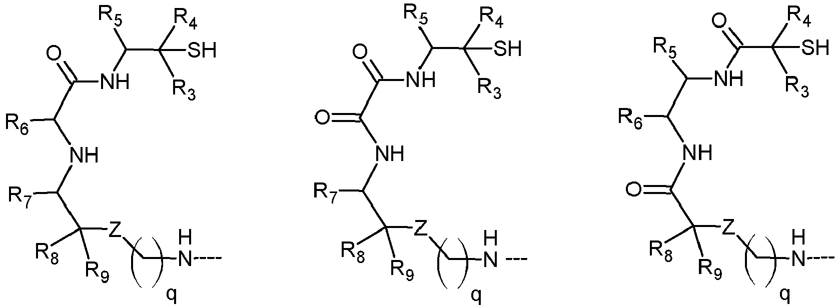 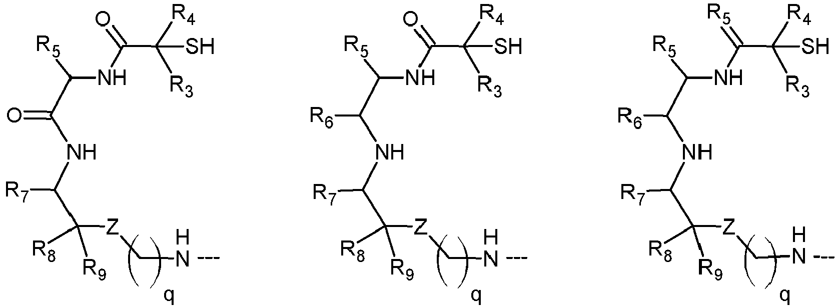 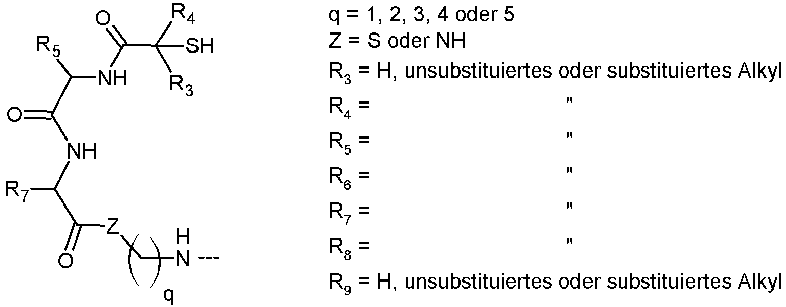 oder
- eine Fluorierungsgruppe Fg gewählt aus der Gruppe umfassend 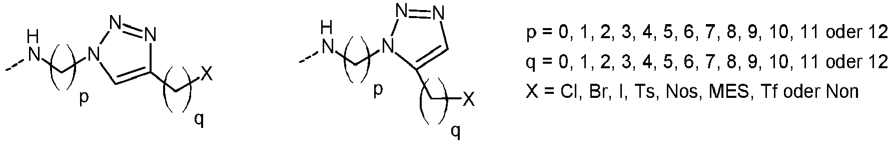 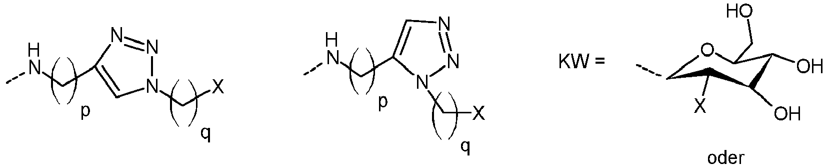 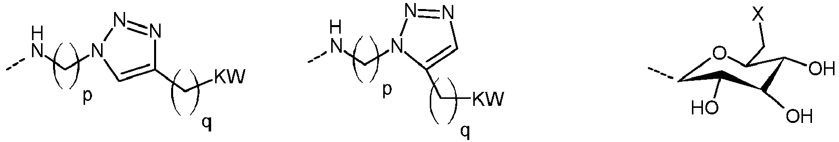 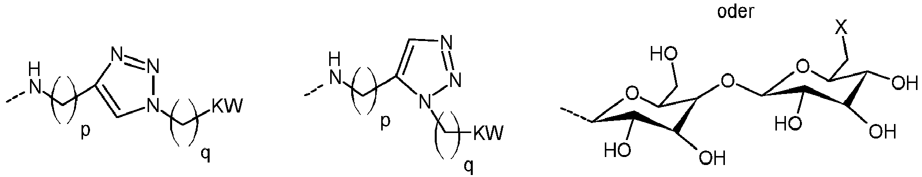 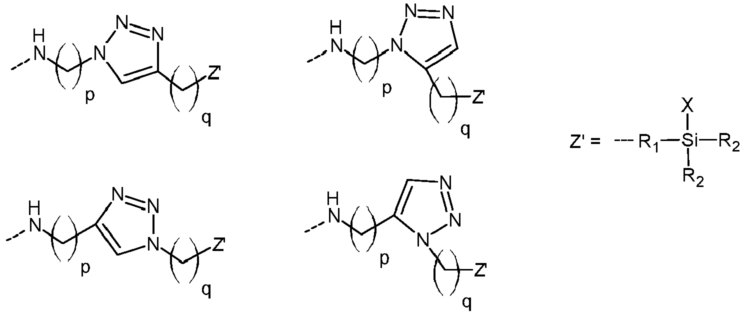 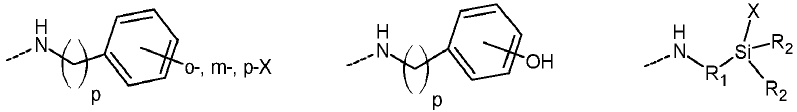 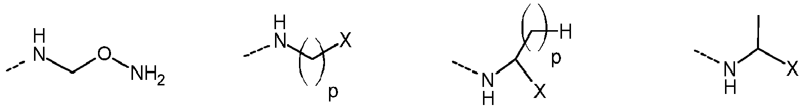 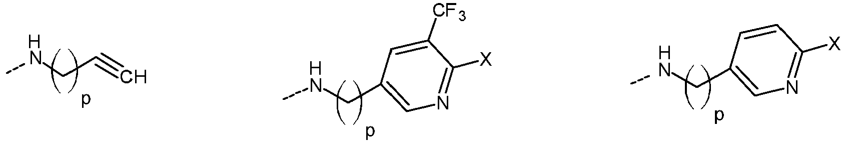 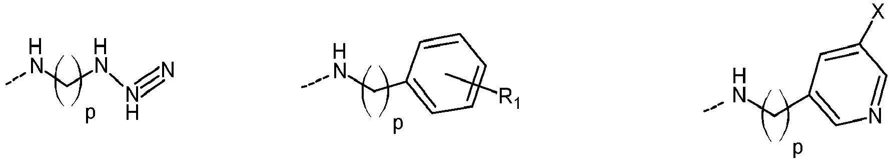 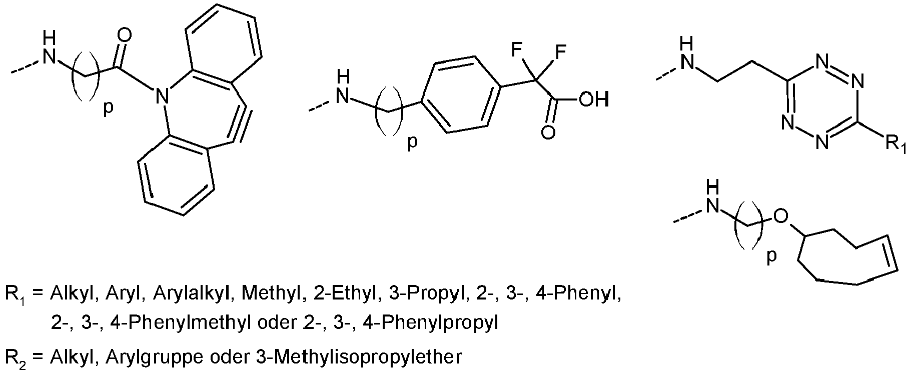
- einen oder zwei Linker L₁ und L₂ , die unabhängig voneinander gewählt sind aus der Gruppe, umfassend Amid-, Carbonsäureamid-, Phosphinat-, Alkyl-, Triazol-, Thioharnstoff-, Ethylen-, Maleimid-Reste, -(CH₂)ₘ- , -(CH₂CH₂O)ₘ- und -(CH₂)ₘNH- mit m = 1, 2, 3, 4, 5, 6, 7, 8, 9 oder 10;
- einen oder mehrere Quadratsäurereste QS 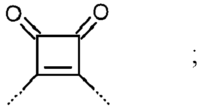
- gegebenenfalls einen, zwei, drei oder vier Spacer Sⱼ mit 1 ≤ j ≤ 4 , die unabhängig voneinander gewählt sind aus der Gruppe, umfassend Amid-, Carbonsäureamid-, Phosphinat-, Alkyl-, Triazol-, Thioharnstoff-, Ethylen-, Maleimid-Reste, -(CH₂)ₙ- , -(CH₂) -CH(COOH)-NH- , -(CH₂CH₂O)ₙ- und -(CH₂)ₙNH- mit n = 1, 2, 3, 4, 5, 6, 7, 8, 9 oder 10; und
- einen oder zwei Targetingvektoren TV₁ und TV₂ , die unabhängig voneinander gewählt sind aus der Gruppe, umfassend Reste von Verbindungen der Struktur [1] bis [41] mit 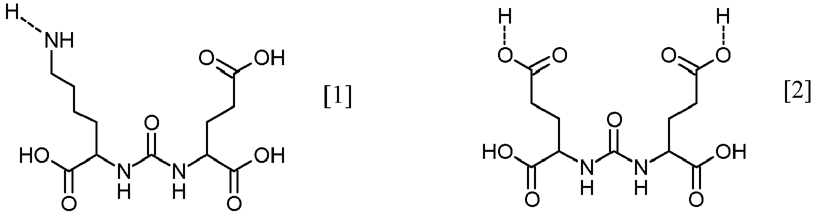 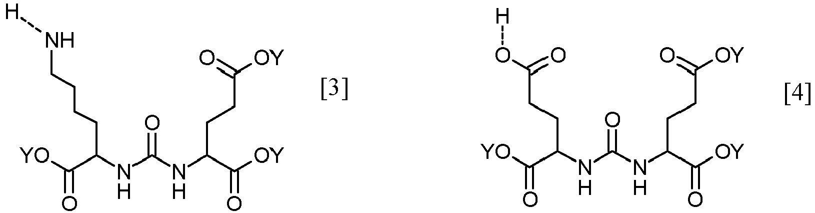 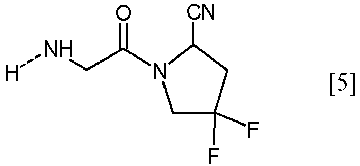 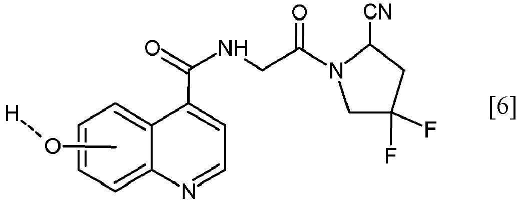 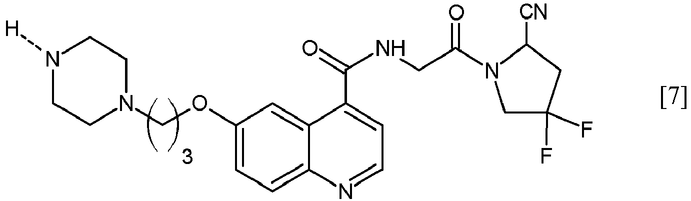 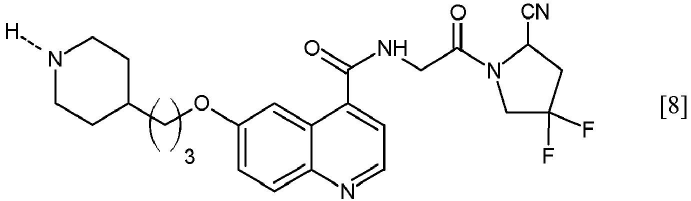 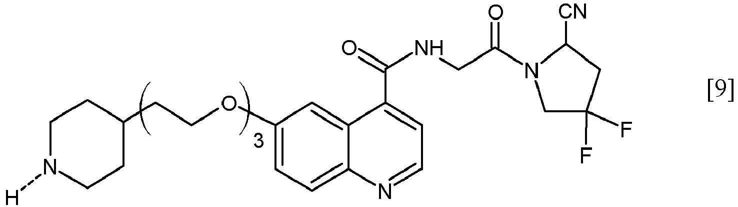 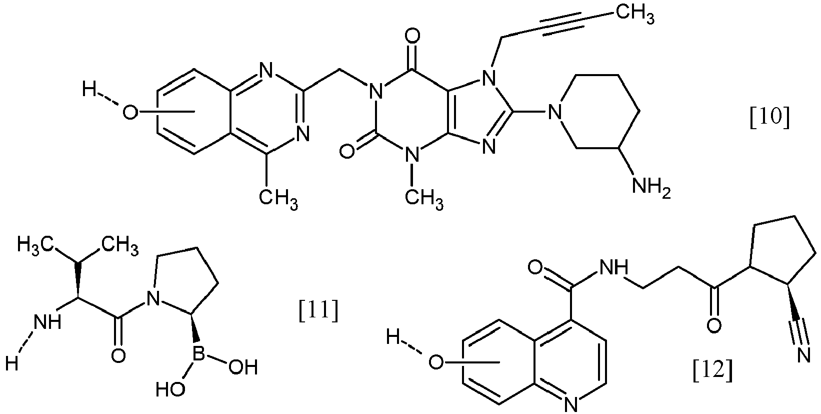 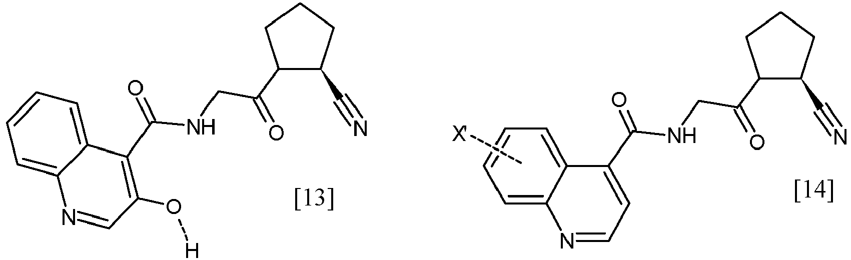 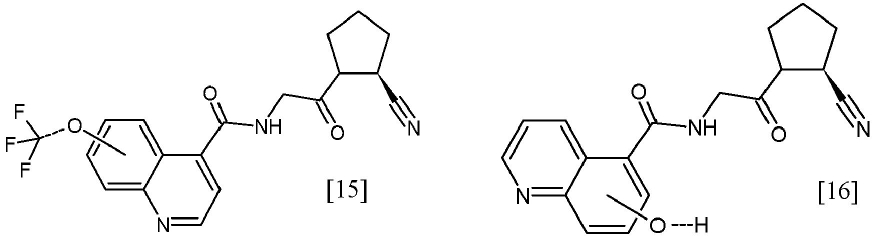 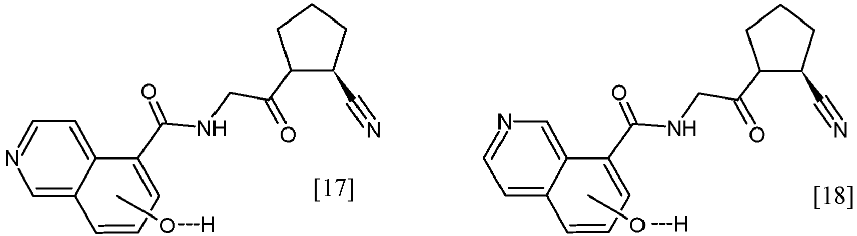 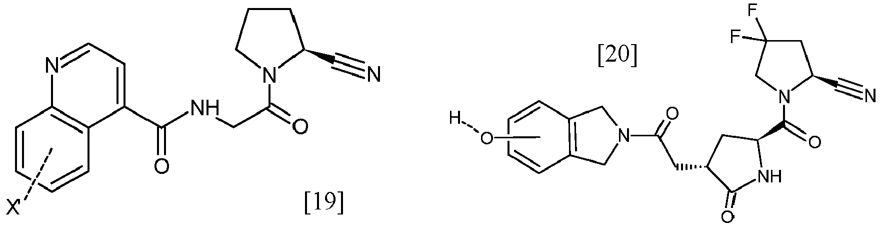 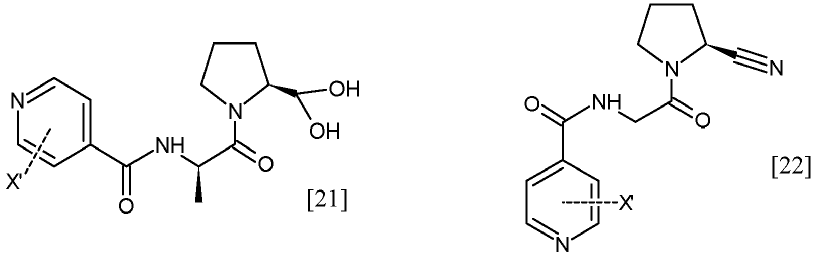 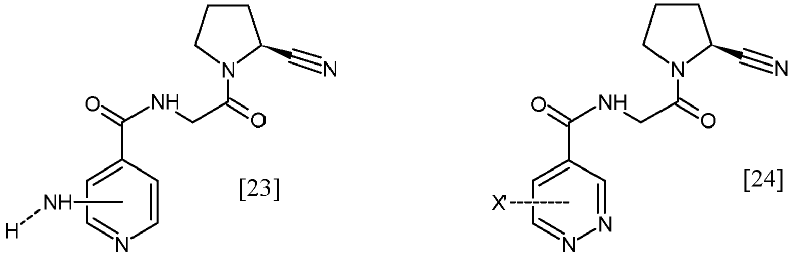 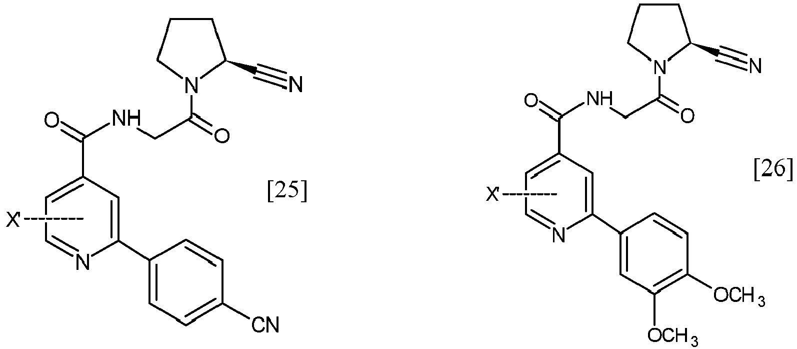 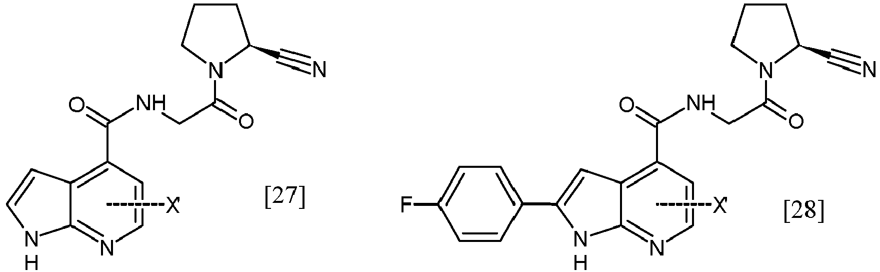 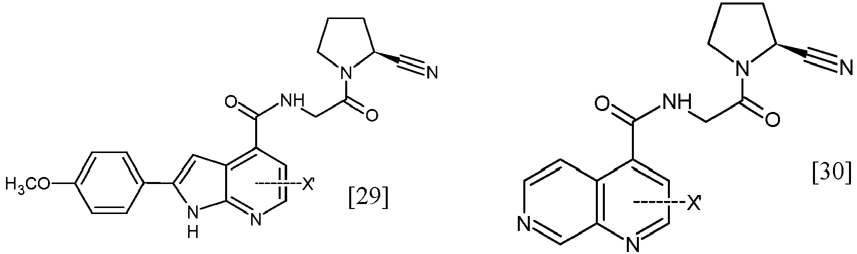 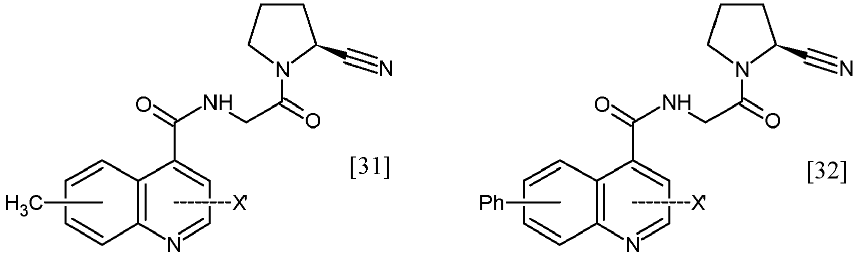 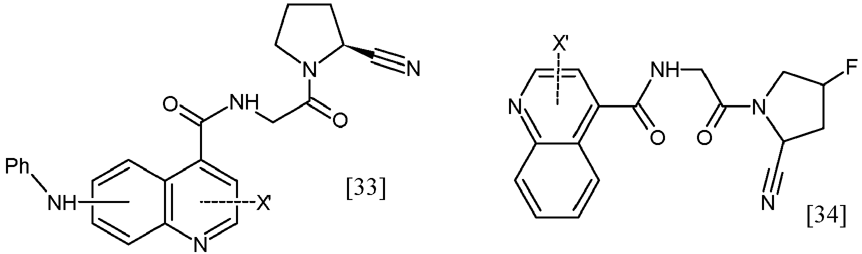 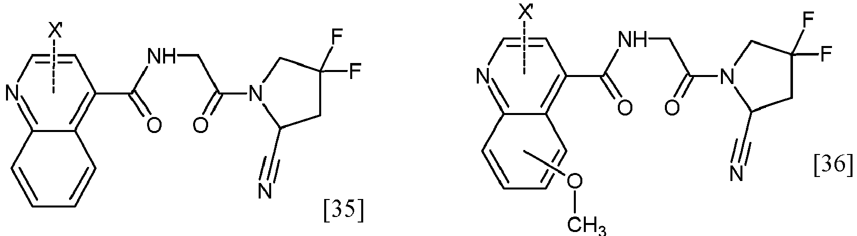 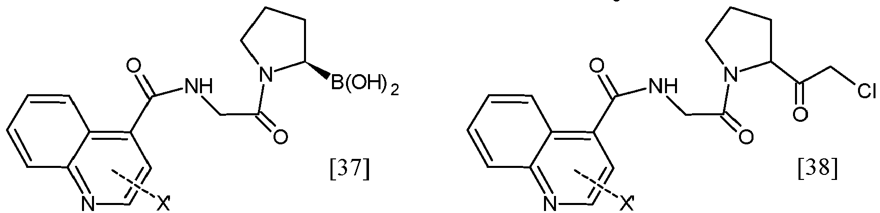 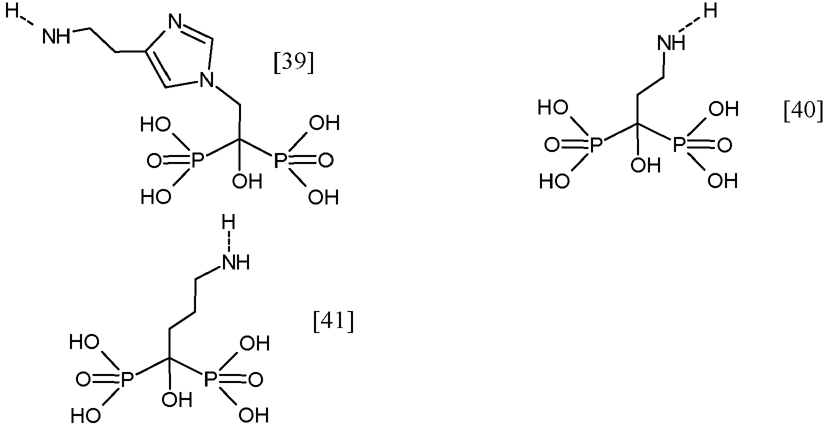 wobei Y eine Schutzgruppe und X' = Cl, Br oder I ist und die gestrichelte Bindung der Targetingvektoren [1]-[41] eine Kopplungsstelle mit einer Abgangsgruppe bezeichnet.

Vorteilhafte Ausführungsformen der nicht anspruchsgemäßen Markierungsvorläufer sind dadurch gekennzeichnet, dass
- der Markierungsvorläufer genau einen Targetingvektor TV₁ enthält;
- der Markierungsvorläufer zwei voneinander verschiedene Targetingvektoren TV₁ und TV₂ mit TV₁ ≠ TV₂ enthält;
- der Markierungsvorläufer zwei gleiche Targetingvektoren TV₁ und TV₂ mit TV₁ = TV₂ enthält;
- die Schutzgruppe Y gewählt ist aus der Gruppe umfassend tert-Butyloxycarbonyl (tert-Butyl), Trialkylsilylgruppen, Trimethylsilyl ( -Si(CH₃)₃ ), Triethylsilyl ( -Si(CH₂CH₃)₃ ), iso-Propyldimethylsilyl ( -Si(CH₃)₂C(CH₃)₂ ), tert-Butyldimethylsilyl ( -Si(CH₃)₂C(CH₃)₃ ) und tert-Butoxydimethylsilyl ( -Si(CH₃)₂OC(CH₃)₃ );
- die Linker L₁ und L₂ gleich sind (L₁ = L₂);
- die Linker L₁ und L₂ voneinander verschieden sind (L₁ ≠ L₂);
- die Spacer S₁ und S₃ gleich sind (S₁ = S₃);
- die Spacer S₁ und S₃ voneinander verschieden sind (S₁ ≠ S₃);
- die Spacer S₂ und S₄ gleich sind (S₂ = S₄); und/oder
- die Spacer S₂ und S₄ voneinander verschieden sind (S₂ ≠ S₄).

Der erfindungsgemäße Markierungsvorläufer ist vorgesehen für die Markierung mit einem Radioisotop gewählt aus der Gruppe, umfassend ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi und ²²⁵Ac beziehungsweise mit ¹⁸F, ¹³¹I oder ²¹¹At.

Dementsprechend betrifft die Erfindung im Weiteren Radiotracer-Verbindungen, die den vorstehend beschriebenen Markierungsvorläufer mit
- einem komplexierten Radioisotop, gewählt aus der Gruppe, umfassend ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb , ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy,¹⁶⁶Ho, ¹⁷⁵Yb, ¹⁷⁷Lu, ¹⁸⁶Re, ¹⁸⁸Re, ²¹³Bi und ²²⁵Ac
umfassen.

Zudem betrifft die Erfindung die Verwendung des vorstehend beschriebenen Markierungsvorläufers zur Herstellung eines Radiopharmakons.

In einer vorteilhaften Ausführungsform wird der vorstehend beschriebene Markierungsvorläufer verwendet für die Herstellung eines mit ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi oder ²²⁵Ac markierten Radiopharmakons.

In einer vorteilhaften Ausführungsform wird der vorstehend beschriebene Markierungsvorläufer verwendet für die Herstellung eines Radiopharmakons für die bildgebende Diagnostik mittels Positronen-Emissions-Tomographie (PET).

In einer vorteilhaften Ausführungsform wird der vorstehend beschriebene Markierungsvorläufer verwendet für die Herstellung eines Radiopharmakons für die bildgebende Diagnostik mittels Einzelphotonen-Emissionscomputertomographie (SPECT).

In einer vorteilhaften Ausführungsform wird der vorstehend beschriebene Markierungsvorläufer verwendet für die Herstellung eines Radiopharmakons für die Behandung von Krebstumoren.

Ein einfaches und effizientes Verfahren für die Synthese nicht anspruchsgemäßer Markierungsvorläufer umfasst die Schritte
- Konjugation eines Chelators Ch oder einer Fluorierungsgruppe Fg mit einem Linker L₁ zu einem Precursor P₁ = Ch-L₁ bzw. P₁ = Fg-L₁ oder Konjugation eines Chelators Ch oder einer Fluorierungsgruppe Fg mit einem Linker L₁ und Quadratsäure QS zu einem Precursor P₂ = Ch-L₁-QS bzw. P₂ = Fg-L₁-QS oder Konjugation eines Chelators Ch mit Linkern L₁ und L₂ zu einem Precursor P₃ = L₂-Ch-L₁ oder Konjugation eines Chelators Ch mit Linkern L₁ , L₂ und Quadratsäure QS zu einem Precursor

   P₄ = QS-L₂-Ch-L₁-QS ;
- gegebenenfalls Konjugation eines Targetingvektors TV₁ mit Quadratsäure QS zu einem Precursor P₅ = TV₁-QS oder Konjugation eines Targetingvektors TV₁ mit Quadratsäure QS und einem Spacer S₂ zu einem Precursor P₆ = TV₁-QS-S₂ oder Konjugation eines Targetingvektors TV₁ mit einem Spacer S₁ zu einem Precursor P₇ = TV₁-S₁ oder Konjugation eines Targetingvektors TV₁ mit einem Spacer S₁ und Quadratsäure QS zu einem Precursor P₈ = TV₁-S₁-QS oder Konjugation eines Targetingvektors TV₁ mit einem Spacer S₁, Quadratsäure QS und einem Spacer S₂ zu einem Precursor P₉ = TV₁-S₁-QS-S₂ ;
- gegebenenfalls Konjugation eines Targetingvektors TV₂ mit Quadratsäure QS zu einem Precursor P₁₀ = TV₂-QS oder Konjugation eines Targetingvektors TV₂ mit Quadratsäure QS und einem Spacer S₄ zu einem Precursor P₁₁ = TV₂-QS-S₄ oder Konjugation eines Targetingvektors TV₂ mit einem Spacer S₃ zu einem Precursor P₁₂ = TV₂-S₃ oder Konjugation eines Targetingvektors TV₂ mit einem Spacer S₃ und Quadratsäure QS zu einem Precursor P₁₃ = TV₂-S₃-QS oder Konjugation eines Targetingvektors TV₂ mit einem Spacer S₃, Quadratsäure QS und einem Spacer S₄ zu einem Precursor P₁₄ = TV₂-S₃-QS-S₄ ;
- Konjugation eines Targetingvektors TV₁ mit dem Precursor P₂ oder Konjugation der Precursor P₁ und P₅ zu einem Markierungsvorläufer der Struktur Ch-L₁-QS-TV₁ bzw. Fg-L₁-QS-TV₁ oder Konjugation der Precursor P₁ und Ps oder P₂ und P₇ zu einem Markierungsvorläufer der Struktur Ch-L₁-QS-S₁-TV₁ bzw. Fg-L₁-QS-S₁-TV₁ oder Konjugation der Precursor P₂ und P₉ zu einem Markierungsvorläufer der Struktur Ch-L₁-QS-S₂-QS-S₁-TV₁ bzw. Fg-L₁-QS-S₂-QS-S₁-TV₁ ; oder
- Konjugation der Precursor P₃, P₅ und P₁₀ zu einem Markierungsvorläufer der Struktur TV₂-QS-L₂-Ch-L₁-QS-TV₁ oder Konjugation der Precursor P₃ , P₈ und P₁₃ oder P₄ , P₇ und P₁₂ zu einem Markierungsvorläufer der Struktur TV₂-S₃-QS-L₂-Ch-L₁-QS-S₁-TV₁ oder Konjugation der Precursor P₄ , P₆ und P₁₁ zu einem Markierungsvorläufer der Struktur TV₂-QS-S₄-QS-L₂-Ch-L₁-QS-S₂-QS-TV₁ oder Konjugation der Precursor P₄ , P₉ und P₁₄ zu einem Markierungsvorläufer der Struktur TV₂-S₃-QS-S₄-QS-L₂-Ch-L₁-QS-S₂-QS-S₁-TV₁;
wobei
- der Chelator Ch gewählt ist aus der Gruppe, umfassend EDTA (Ethylendiamintetraacetat), EDTMP (Diethylentriaminpenta(methylenphosphonsäure)), DTPA (Diethylentriaminpentaacetat) und dessen Derivate, DOTA (Dodeca-1,4,7,10-tetraamin-tetraacetat), DOTAGA (2-(1,4,7,10-Tetraazacyclododecan-4,7,10)-pentandisäure) und anderen DOTA-Derivaten, TRITA (Trideca-1,4,7,10-tetraamin-tetraacetat), TETA (Tetradeca-1,4,8,11-tetraamin-tetra-acetat) und dessen Derivate, NOTA (Nona-1,4,7-triamin-triacetat) und dessen Derivate wie beispielsweise NOTAGA (1,4,7-triazacyclononan,1-glutarsäure,4,7-acetat), NOPO (1,4,7-triazacyclononan-1,4-bis[methylen(hydroxymethyl)phosphinsäure]-7-[methylen(2-carboxyethyl)phosphinsäure]), PEPA (Pentadeca-1,4,7,10,13-pentaaminpentaacetat), HEHA (Hexadeca-1,4,7,10,13,16-hexaamin-hexaacetat) und dessen Derivate, HBED (Hydroxybenzyl-ethylen-diamin) und dessen Derivate, DEDPA und dessen Derivate, wie H₂DEDPA (1,2-[[6-(carboxylat-)pyridin-2-yl]methylamin]ethan), DFO (Deferoxamin) und dessen Derivate, Trishydroxypyridinon (THP) und dessen Derivate wie YM103, TRAP (Triazacyclononan-phosphinsäure), TEAP (Tetraazycyclodecan-phosphinsäure) und dessen Derivate, AAZTA (6-Amino-6-methylperhydro-1,4-diazepin-N,N,N',N'-tetraacetat) und Derivate wie DATA ((6-Pentansäure)-6-(amino)methyl-1,4-diazepin triacetat); SarAr (1-N-(4-aminobenzyl)-3,6,10,13,16,19-hexaazabicyclo[6.6.6]-eicosan-1,8-diamin) und Salze davon, Aminothiole und deren Derivate des Typs 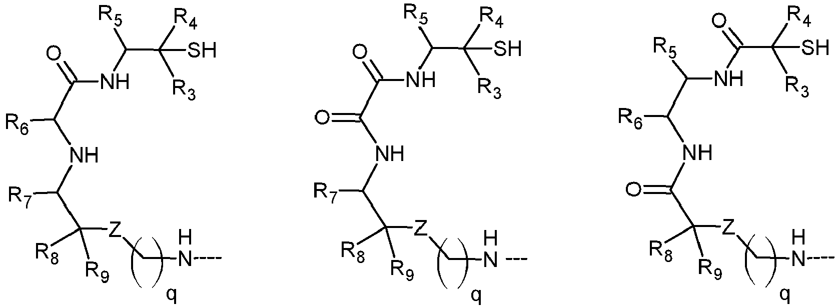 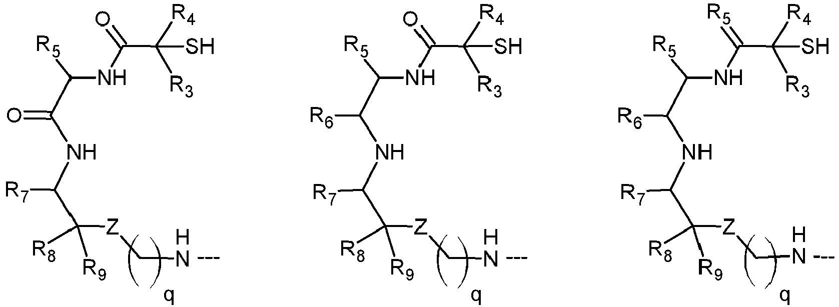 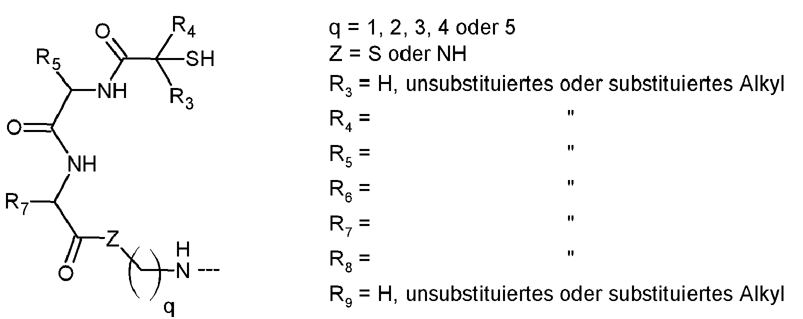
- die Fluorierungsgruppe Fg gewählt ist aus der Gruppe umfassend 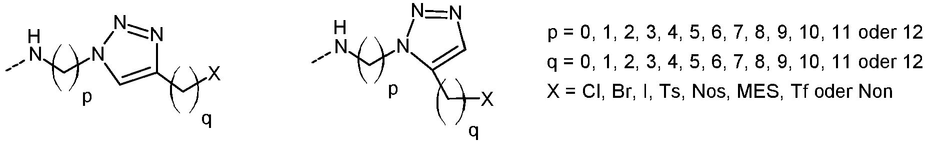 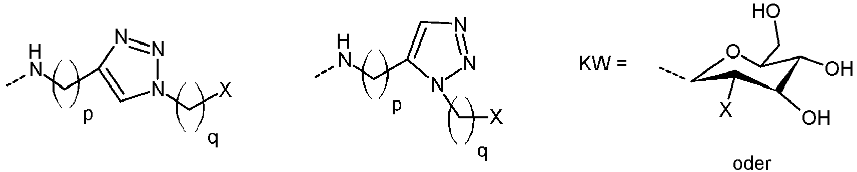 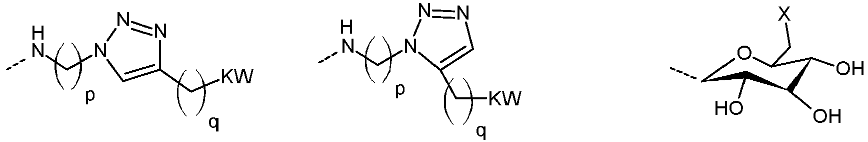 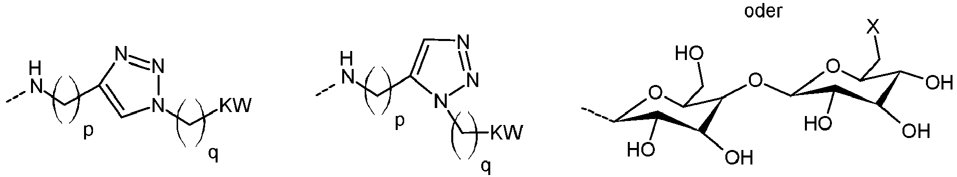 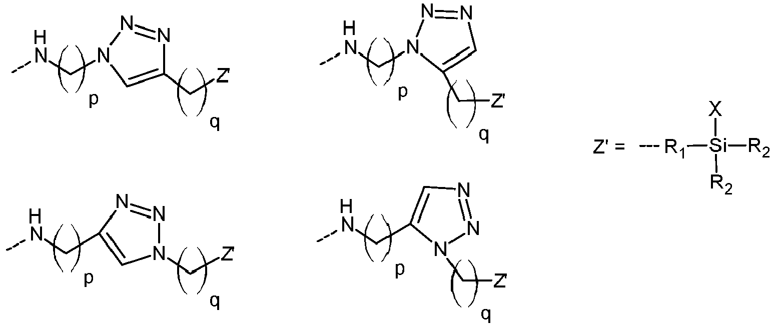 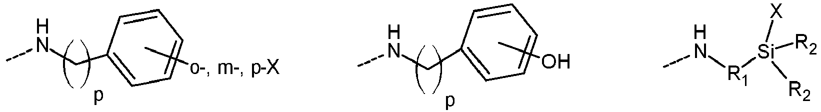 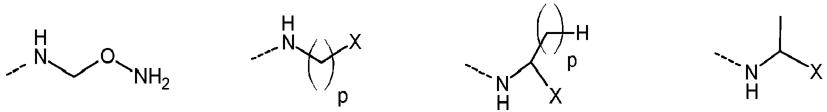 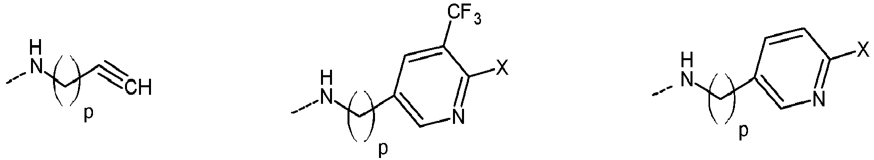 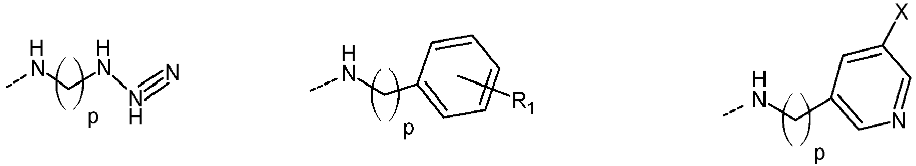 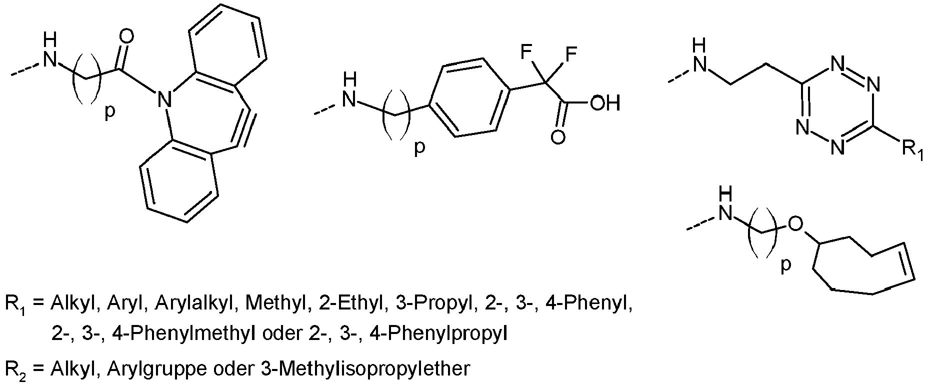
- die Linker L₁ und L₂ unabhängig voneinander gewählt sind aus der Gruppe, umfassend Amid-, Carbonsäureamid-, Phosphinat-, Alkyl-, Triazol-, Thioharnstoff-, Ethylen-, Maleimid-Reste, -(CH₂)ₘ- , -(CH₂CH₂O)ₘ- und -(CH₂)ₘNH- mit m = 1, 2, 3, 4, 5, 6, 7, 8, 9 oder 10;
- die Spacer Sⱼ mit 1 ≤ j ≤ 4 unabhängig voneinander gewählt sind aus der Gruppe, umfassend Amid-, Carbonsäureamid-, Phosphinat-, Alkyl-, Triazol-, Thioharnstoff-, Ethylen-, Maleimid-Reste, -(CH₂)n- , -(CH₂)-CH(COOH)-NH- , -(CH₂CH₂O)ₙ- und -(CH₂)ₙNH- mit n = 1, 2, 3, 4, 5, 6, 7, 8, 9 oder 10;
und
- die Targetingvektoren TV₁ und TV₂ unabhängig voneinander gewählt sind aus der Gruppe, umfassend Verbindungen der Struktur [1] bis [41] mit 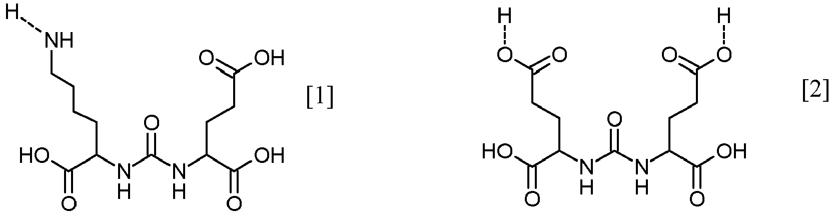 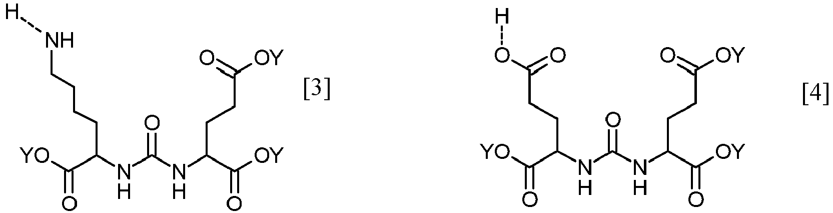 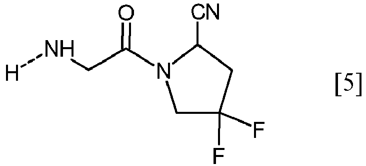 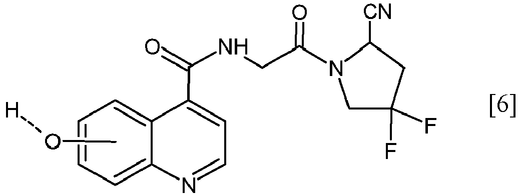 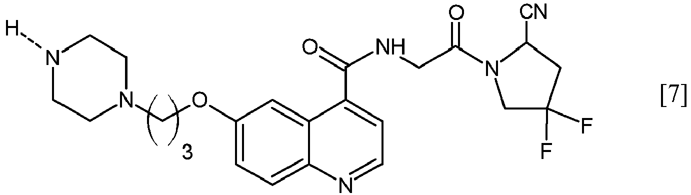 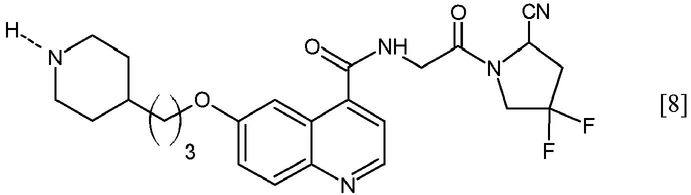 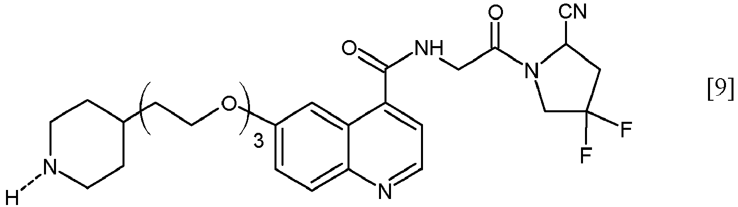 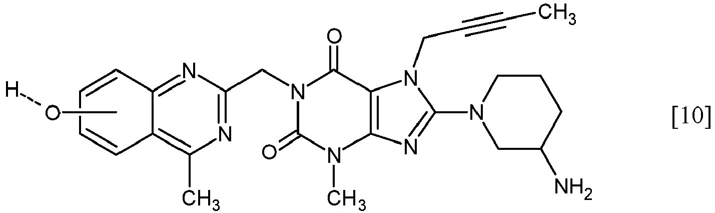 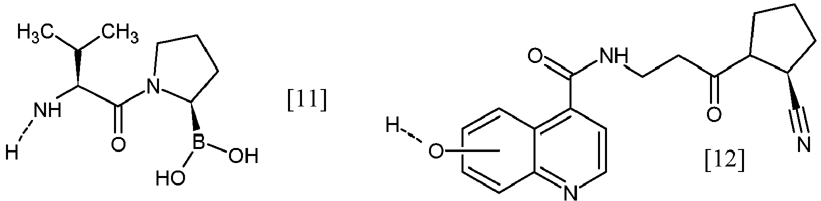 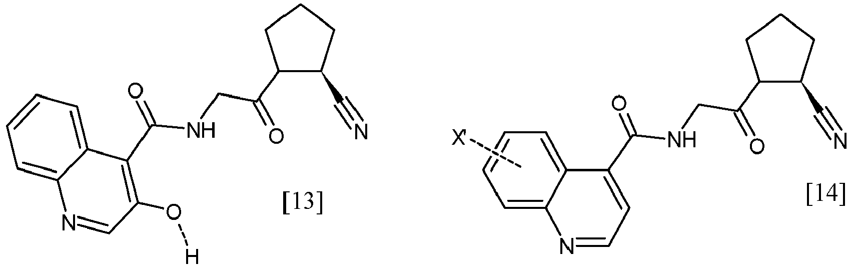 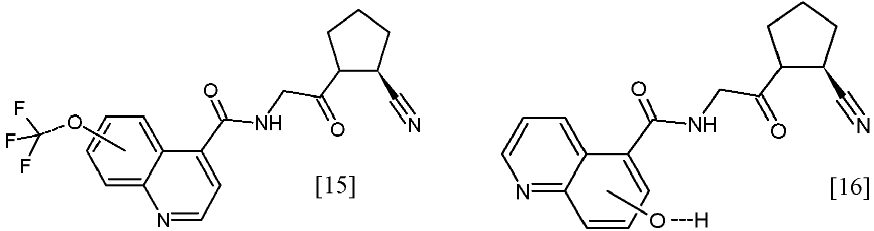 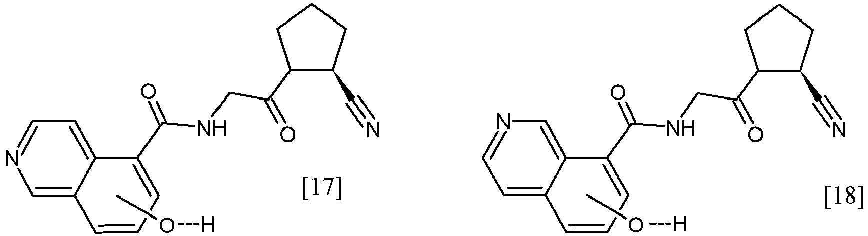 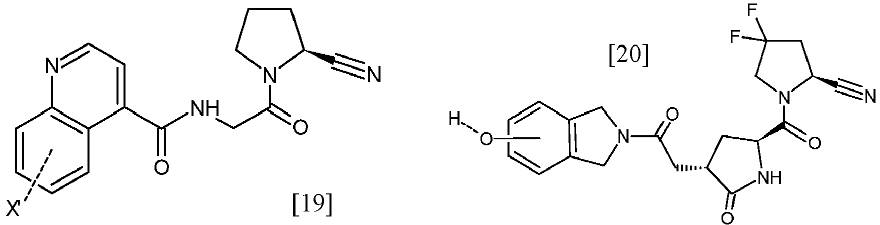 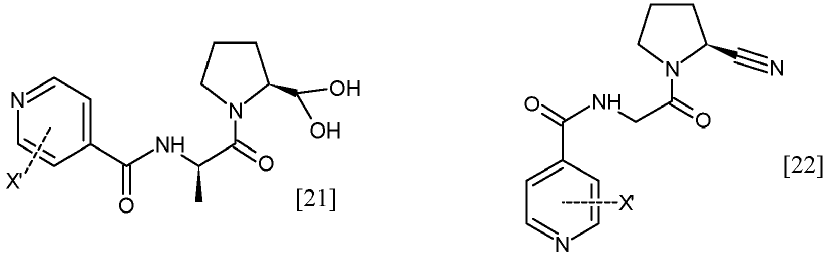 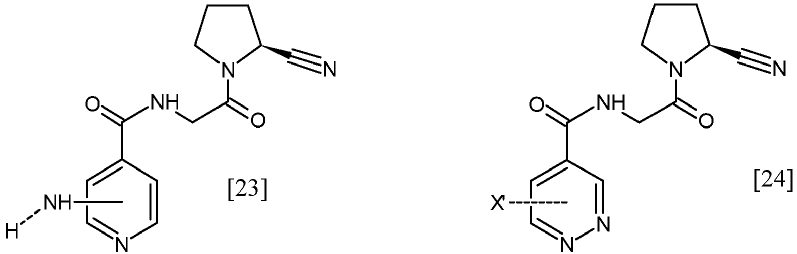 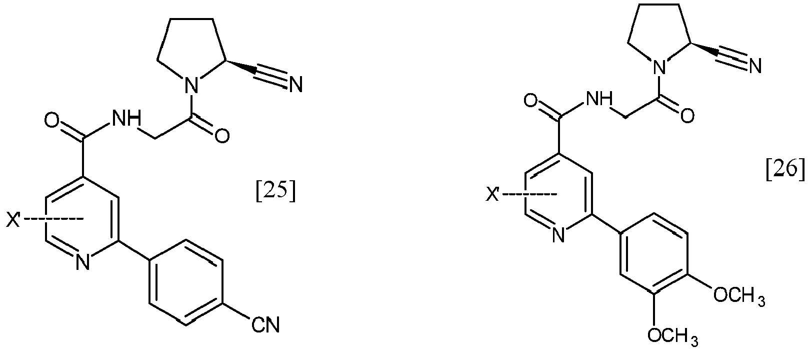 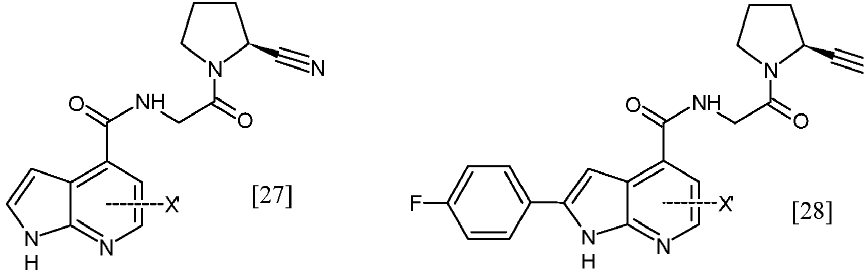 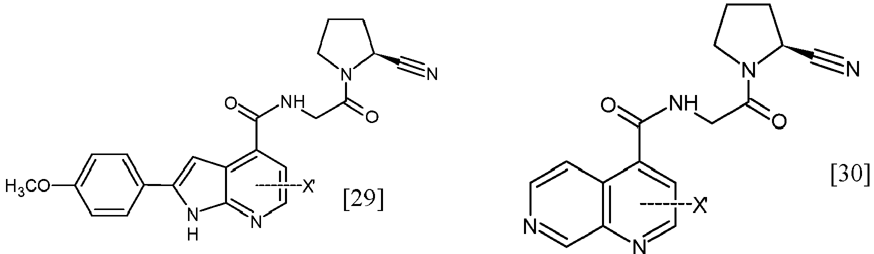 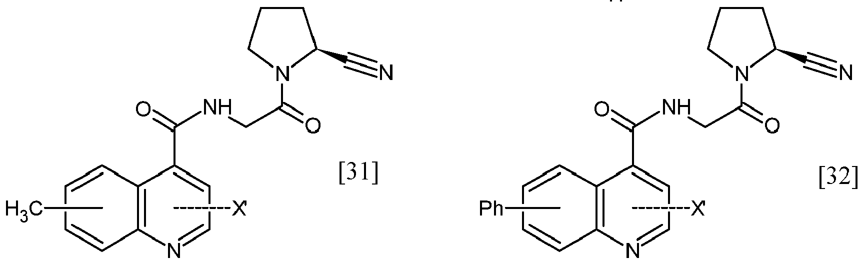 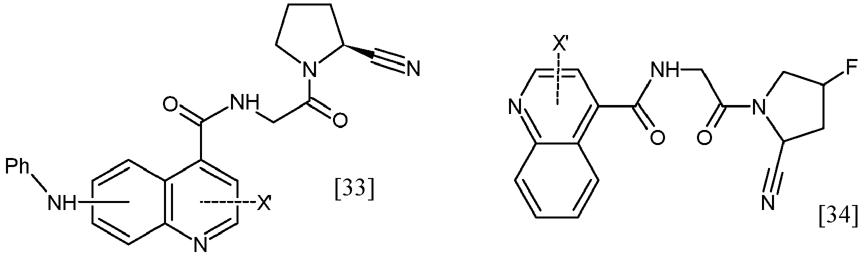 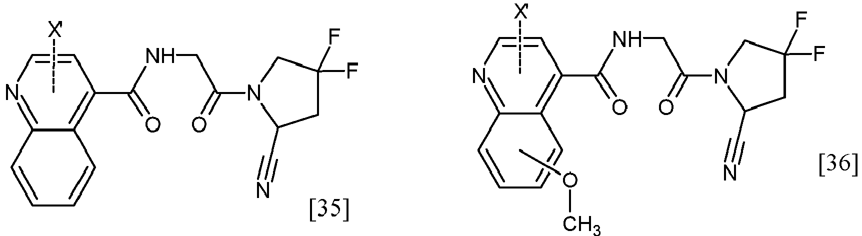 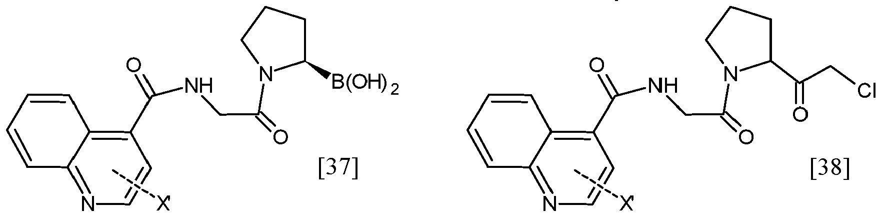 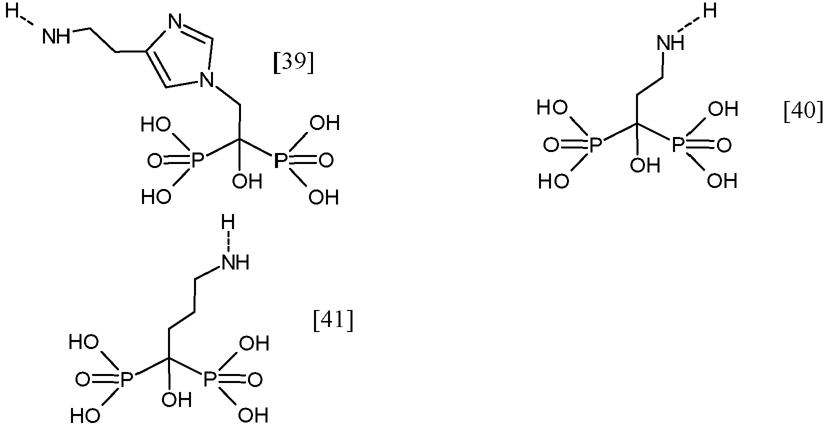 wobei Y eine Schutzgruppe und X' = Cl, Br oder I ist und die gestrichelte Bindung der Targetingvektoren [1]-[41] eine Kopplungsstelle mit einer Abgangsgruppe bezeichnet.

Vorteilhafte Ausführungsformen des nicht anspruchsgemäßen Verfahrens sind dadurch gekennzeichnet, dass
- die Targetingvektoren TV₁ und TV₂ voneinander verschieden sind (TV₁ ≠ TV₂);
- die Targetingvektoren TV₁ und TV₂ gleich sind (TV₁ = TV₂);
- die Schutzgruppe Y gewählt ist aus der Gruppe umfassend tert-Butyloxycarbonyl (tert-Butyl), Trialkylsilylgruppen, Trimethylsilyl ( -Si(CH₃)₃ ), Triethylsilyl ( -Si(CH₂CH₃)₃ ), iso-Propyldimethylsilyl ( -Si(CH₃)₂C(CH₃)₂ ), tert-Butyldimethylsilyl ( -Si(CH₃)₂C(CH₃)₃ ) und tert-Butoxydimethylsilyl ( -Si(CH₃)₂OC(CH₃)₃ );
- die Linker L₁ und L₂ gleich sind (L₁ = L₂);
- die Linker L₁ und L₂ voneinander verschieden sind (L₁ ≠ L₂);
- die Spacer S₁ und S₃ gleich sind (S₁ = S₃);
- die Spacer S₁ und S₃ voneinander verschieden sind (S₁ ≠ S₃);
- die Spacer S₂ und S₄ gleich sind (S₂ = S₄); und/oder
- die Spacer S₂ und S₄ voneinander verschieden sind (S₂ ≠ S₄).

Die Fluorierungsgruppe Fg umfasst eine Abgangsgruppe X für die Markierung mit einem der Radioisotope ¹⁸F, ¹³¹I oder ²¹¹At. Die Abgangsgruppe X ist gleich einem Rest von Brom (Br), Chlor (Cl), Iod (I), Tosyl (-SO₂-C₆H₄-CH₃; abgekürzt "Ts"), Nosylat bzw. Nitrobenzolsulfonat (-OSO₂-C₆H₄-NO₂; abgekürzt "Nos"), 2-(N-Morpholino)ethansulfonsäure (-SO₃-(CH₂)₂-N(CH₂)₄O; abgekürzt "MES"), Triflat bzw. Trifluormethansulfonyl (-SO₂CF₃; abgekürzt "Tf') oder Nonaflat (-OSO₂-C₄F₉; abgekürzt "Non").

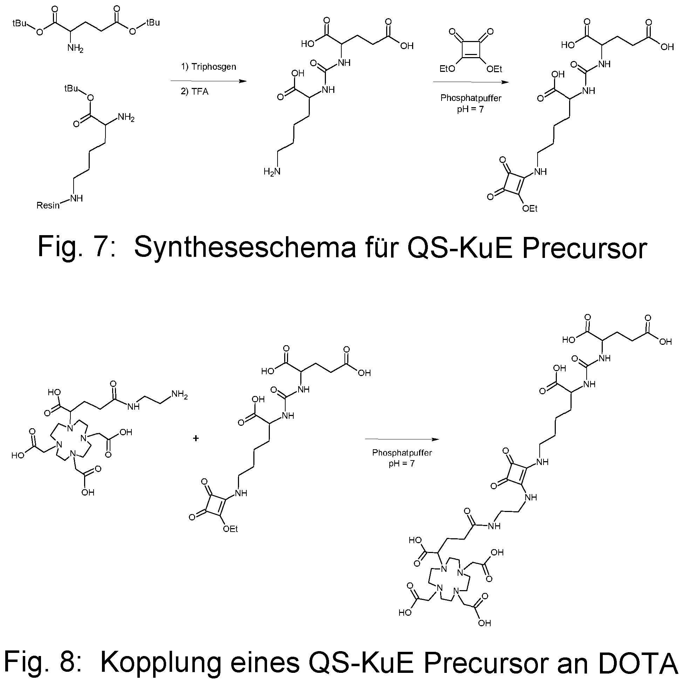

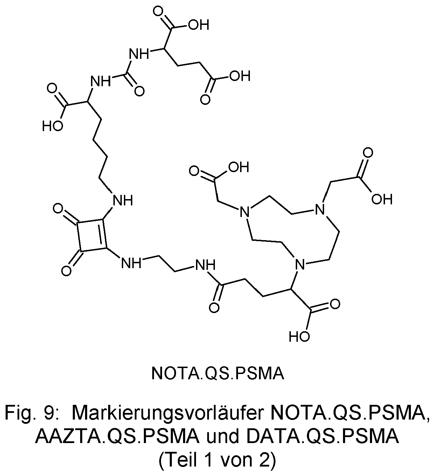

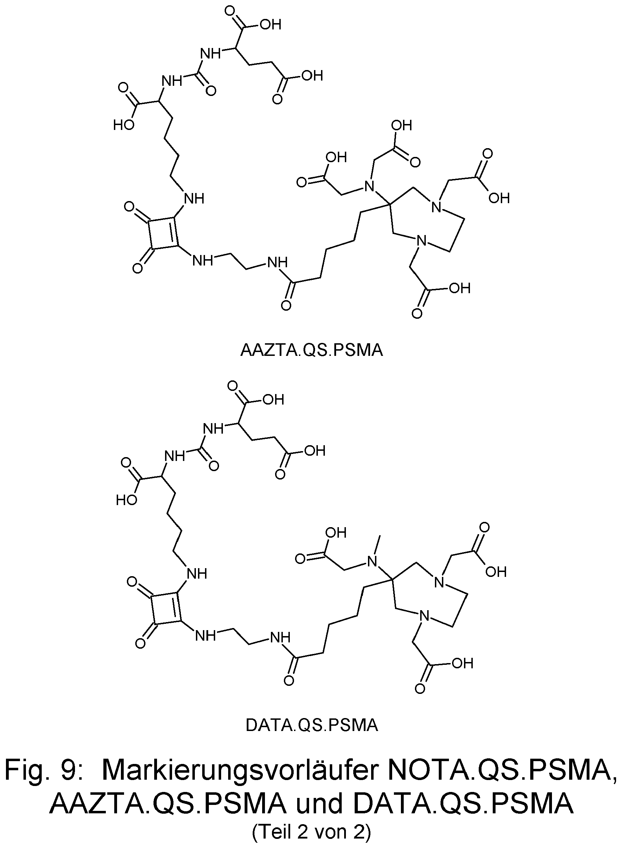

Im Rahmen der vorliegenden Erfindung werden folgende Bezeichnungen bzw. Abkürzungen verwendet:
- PSMA: prostataspezifisches Membranantigen;
- FAP: Fibroblasten-Aktivierungs-Protein;
- FPPS: Farnesyl-Pyrophosphat-Synthase;
- 2-PMPA: 2-Phosphonomethyl-Glutarsäure;
- KuE: L-Lysin-Urea-L-Glutamat;
- DOTA.QS.PSMA: Markierungsvorläufer, insbesondere mit Strukturformel gemäß Fig. 8, umfassend DOTA (Dodeca-1,4,7,10-tetraamin-tetraacetat) als Chelator, der über einen oder zwei Linker, Quadratsäuregruppen und Spacer mit einem oder zwei PSMA-Targetingvektoren mit Strukturformel [1], [2], [3] und/oder [4] gekoppelt ist;
- NOTA.QS.PSMA: Markierungsvorläufer, insbesondere mit Strukturformel gemäß Fig. 9, umfassend NOTA (Nona-1,4,7-triamin-triacetat) als Chelator, der über einen oder zwei Linker, Quadratsäuregruppen und Spacer mit ein oder zwei PSMA-Targetingvektoren mit Strukturformel [1], [2], [3] und/oder [4] gekoppelt ist;
- AAZTA.QS.PSMA: Markierungsvorläufer, insbesondere mit Strukturformel gemäß Fig. 9, umfassend AAZTA als Chelator, der über einen oder zwei Linker, Quadratsäuregruppen und Spacer mit ein oder zwei PSMA-Targetingvektoren mit Strukturformel [1], [2], [3] und/oder [4] gekoppelt ist;
- DATA.QS.PSMA: Markierungsvorläufer, insbesondere mit Strukturformel gemäß Fig. 9, umfassend DATA als Chelator, der über einen oder zwei Linker, Quadratsäuregruppen und Spacer mit einem oder zwei PSMA-Targetingvektoren mit Strukturformel [1], [2], [3] und/oder [4] gekoppelt ist;
- DOTAGA.QS.PSMA: Markierungsvorläufer, umfassend DOTAGA (2-(1,4,7,10-Tetraazacyclododecan-4,7,10)-pentandisäure) als Chelator, der über einen oder zwei Linker, Quadratsäuregruppen und Spacer mit einem oder zwei PSMA-Targetingvektoren mit Strukturformel [1], [2], [3] und/oder [4] gekoppelt ist;
- NOTAGA.QS.PSMA: Markierungsvorläufer, umfassend NOTAGA (1,4,7-triazacyclononan,1-glutarsäure,4,7-acetat) als Chelator, der über einen oder zwei Linker, Quadratsäuregruppen und Spacer mit einem oder zwei PSMA-Targetingvektoren mit Strukturformel [1], [2], [3] und/oder [4] gekoppelt ist;
- TRAP.QS.PSMA: Markierungsvorläufer, umfassend TRAP (Triazacyclononan-phosphinsäure) als Chelator, der über einen oder zwei Linker, Quadratsäuregruppen und Spacer mit einem oder zwei PSMA-Targetingvektoren mit Strukturformel [1], [2], [3] und/oder [4] gekoppelt ist.
- NOTA.QS.PAM: Markierungsvorläufer, umfassend NOTA als Chelator, der über einen oder zwei Linker, Quadratsäuregruppen und Spacer mit einem oder zwei Pamidronat-Targetingvektoren gemäß Strukturformel [40] gekoppelt ist.

Weitere im Rahmen der Erfindung verwendete Abkürzungen korrespondieren zu den vorstehenden Abkürzungen, wobei ein anderer Chelator, eine andere Fluorierungsgruppe und/oder ein anderer Targetingvektor - insbesondere ein Targetingvektor für FAP gemäß den Strukturformeln [5] bis [41] - in analoger Weise durch seine jeweilige Abkürzung oder Akronym bezeichnet ist. Beispielsweise werden analoge Derivate, die zum Targeting von Farnesyl-Pyrophosphat-Synthase (FPPS) in Knochenmetastasen eingesetzt werden, je nach Art des Bisphosphonats mit "PAM" für Pamidronat und "ZOL" für Zoledronat abgekürzt.

Ein nicht anspruchsgemäßer Markierungsvorläufer umfasst gegebenenfalls einen oder mehrere Spacer Sⱼ mit 1 ≤ j ≤ 4 , d. h. einen Spacer S₁, zwei Spacer S₁ und S₂, drei Spacer S₁, S₂ und S₃ oder vier Spacer S₁, S₂, S₃ und S₄.

In den Strukturformeln [1]-[41] der Targetingvektoren sind die für die Konjugation mit einer Quadratsäuregruppe oder einem Spacer S₁ oder S₂ vorgesehenen Bindungen gestrichelt dargestellt. Die über die gestrichelte Bindung konjugierte Gruppe ist eine Abgangsgruppe, die bei der Kopplung des Targetingvektors mit der Quadratsäuregruppe oder dem Spacer S₁ oder S₂ abgespalten wird.

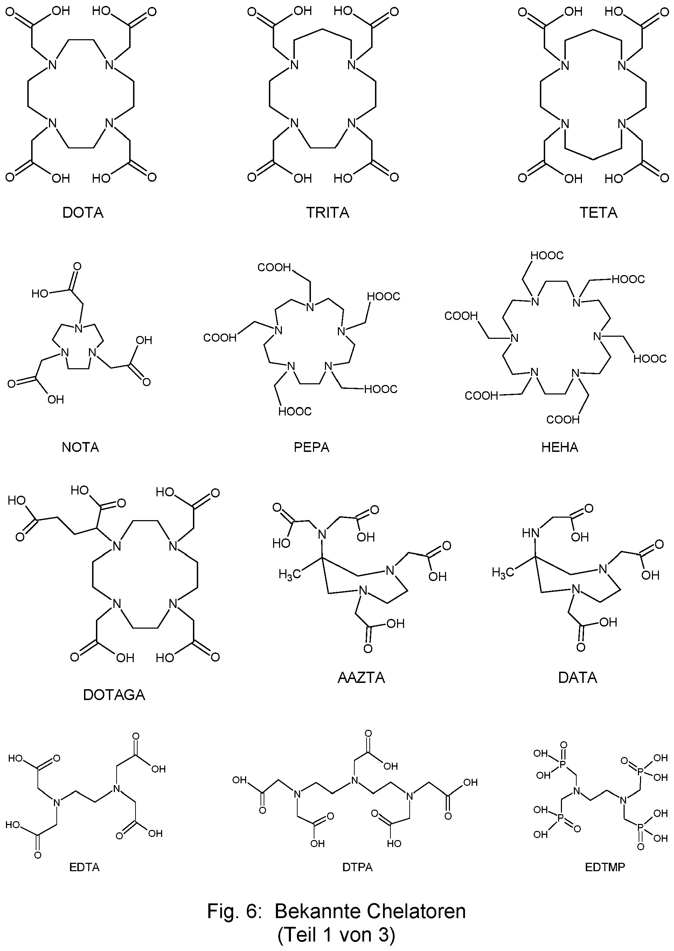

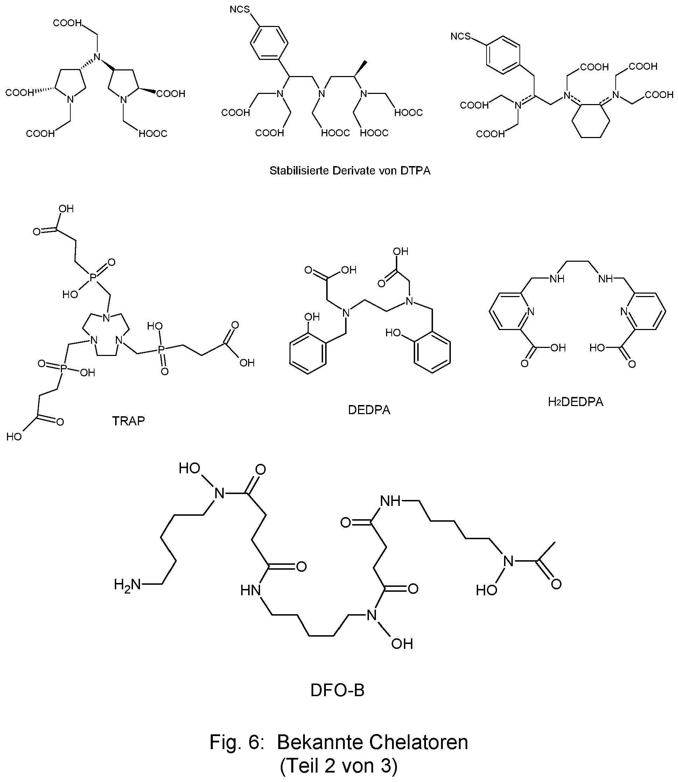

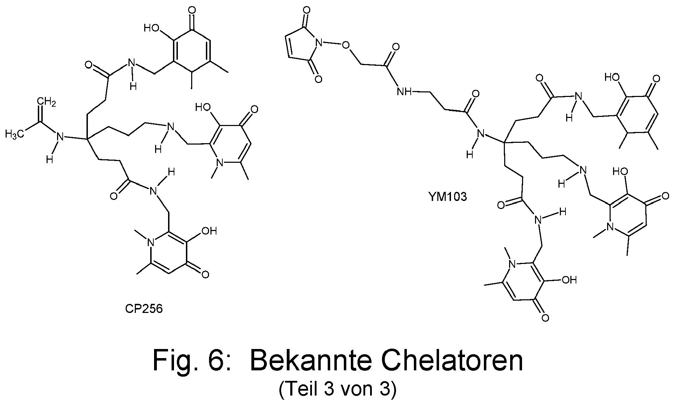

Nachfolgend wird die Erfindung anhand von Figuren und Beispielen näher erläutert. Fig. 6 zeigt die Struktur einiger bekannter Chelatoren Ch (Fig. 6: Bekannte Chelatoren).

### Nicht anspruchsgemäßes Beispiel 1: Synthesestrategie für PSMA-Markierungsvorläufer

Bei der Synthese der Markierungsvorläufer werden vorzugsweise Quadratsäure-Diester eingesetzt. Dadurch ist eine Vielzahl, zum Teil sehr komplexer Markierungsvorläufer mittels einfacher Reaktionsverfahren darstellbar. Quadratsäure-Diester zeichnen sich durch ihre selektive Reaktivität mit Aminen aus, so dass bei der Kopplung von Chelatoren, Linkern, Spacern und Targetingvektoren keine Schutzgruppen benötigt werden. Zudem ist die Kopplung über den pH-Wert steuerbar.

Zunächst wird ein Targetingvektor für PSMA synthetisiert (siehe Fig. 7) und nach Aufreinigung in wässrigem Medium bei pH = 7 mit Quadratsäurediester umgesetzt zu einer prostethischen Gruppe bzw. einem Precursor für die Kopplung mit einem Chelator (siehe Fig. 8) (Fig. 7: Syntheseschema für QS-KuE Precursor).

Im Fall eines Targetingvektors für PSMA wird z. B. mittels eines bekannten Verfahrens der PSMA-Inhibitor L-Lysin-Urea-L-Glutamat (KuE) synthetisiert. Hierbei wird an eine Festphase, insbeondere ein Polymerharz gebundenes und mit tert-Butyloxycarbonyl (tert-Butyl) geschütztes Lysin mit zweifach tert-Butyl-geschützter Glutaminsäure umgesetzt. Nach Aktivierung der geschützten Glutaminsäure durch Triphosgen und der Kopplung an das festphasengebundene Lysin wird L-Lysin-Urea-L-Glutamat (KuE) mittels TFA abgespalten und zugleich vollständig entschützt. Das Produkt kann anschließend mittels semipräparativer HPLC von freiem Lysin getrennt werden. Die auf Lysin bezogene Ausbeute der vorstehenden Reaktion ist größer als 50% (Fig. 8: Kopplung eines QS-KuE Precursor an DOTA).

Der QS-KuE Precursor wird in Phosphatpuffer bei einem pH-Wert von 9 mit dem Chelator DOTA zu dem Markierungsvorläufer DOTA.QS.PSMA konjugiert.

Für die Radiomarkierung der PSMA-Markierungsvorläufer wurde ⁶⁸Ga mit 0,6 M HCl von einem iThemba Ge/Ga-Generator eluiert und mittels wässriger Ethanol-Elution über eine Kationentauschersäule aufbereitet. Die Radiomarkierung erfolgt je nach Chelator bei pH-Werten zwischen 3,5 und 5,5 und Temperaturen zwischen 25°C und 95°C. Der Reaktionsverlauf wurde mittels HPLC und IPTC aufgezeichnet, um die kinetischen Parameter der Reaktion zu ermitteln.

### Nicht anspruchsgemäßes Beispiel 2: Markierungsvorläufer NOTA.QS.PSMA, AAZTA.QS.PSMA und DATA.QS.PSMA

Mittels einer Synthese gemäß der in Beispiel 1 beschriebenen Strategie wurden unter Verwendung der Chelatoren NOTA, AAZTA und DATA anstelle von DOTA die in Fig. 9 wiedergebenen Markierungsvorläufer NOTA.QS.PSMA, AAZTA.QS.PSMAund DATA.QS.PSMA dargestellt (Fig. 9: Markierungsvorläufer NOTA.QS.PSMA, AAZTA.QS.PSMA und DATA.QS.PSMA).

### Nicht anspruchsgemäßes Beispiel 3: PSMA-Markierungsvorläufer für Radiohalogenide

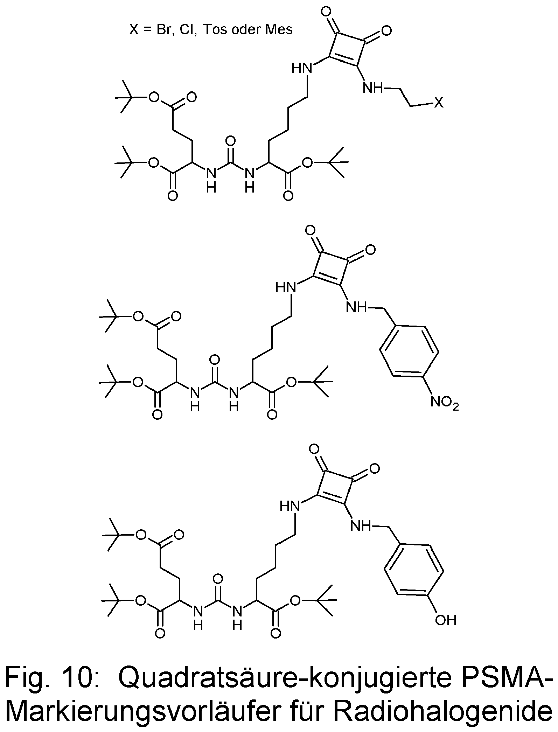

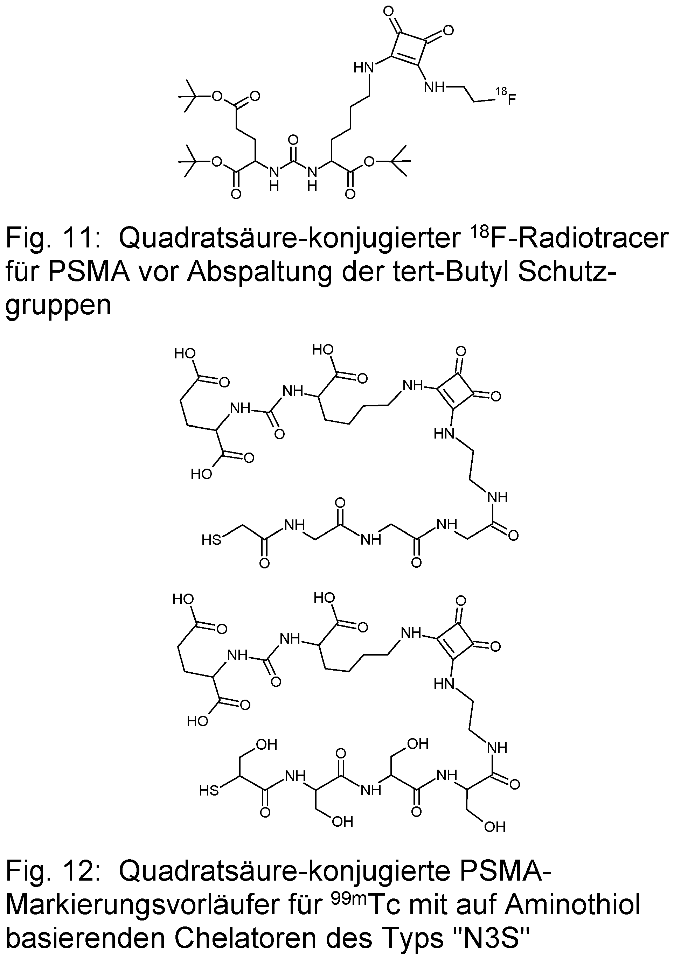

Mittels geringfügig abgewandelter Reaktionen nach dem Syntheseschema der Fig. 7 wurden die in Fig. 10 wiedergegebenen PSMA-Markierungsvorläufer für die Radiomarkierung mit Halogenidisotopen, wie ¹⁸F und ¹³¹I dargestellt. Ein entsprechender mit ¹⁸F markierter Radiotracer ist in Fig. 11 wiedergegeben (Fig. 10: Quadratsäure-konjugierte PSMA-Markierungsvorläufer für Radiohalogenide; Fig. 11: Quadratsäure-konjugierter ¹⁸F-Radiotracer für PSMA vor Abspaltung der tert-Butyl Schutzgruppen).

### Nicht anspruchsgemäßes Beispiel 4: PSMA-Markierungsvorläufer für ^{99m}Tc

Mittels einer Synthese gemäß der in Beispiel 1 beschriebenen Strategie wurden die in Fig. 12 wiedergebenen PSMA-Markierungsvorläufer für die Radiomarkierung mit dem Isotop ^{99m}Tc dargestellt (Fig. 12: Quadratsäure-konjugierte PSMA-Markierungsvorläufer für ^{99m}Tc mit auf Aminothiol basierenden Chelatoren des Typs "N₃S").

### Beispiel 5: Synthesestrategie für FAP-Markierungsvorläufer

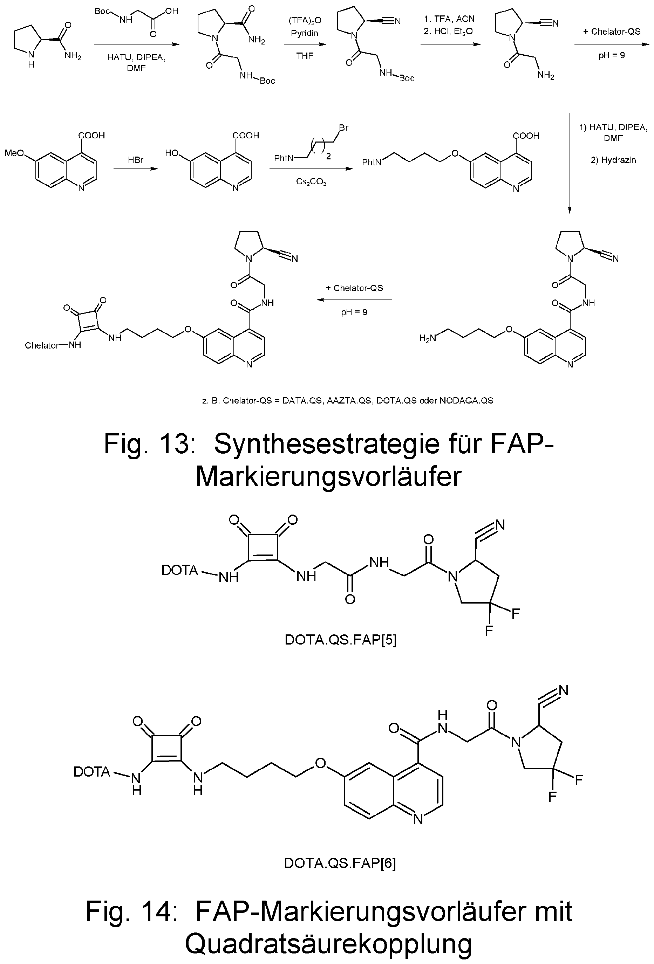

(Fig. 13: Synthesestrategie für FAP-Markierungsvorläufer)

Fig. 14 zeigt zwei FAP-Markierungsvorläufer, die gemäß der in Fig. 13 gezeigten Synthesestrategie dargestellt wurden (Fig. 14: FAP-Markierungsvorläufer mit Quadratsäurekopplung).

### Nicht anspruchsgemäßes Beispiel 6: QS als Komplexierungshelfer

Für die klinische Anwendung ist es sehr wichtig, dass die Komplexierung bei niedriger Temperatur effizient erfolgt. Quadratsäuren komplexieren freie Metalle und können somit das

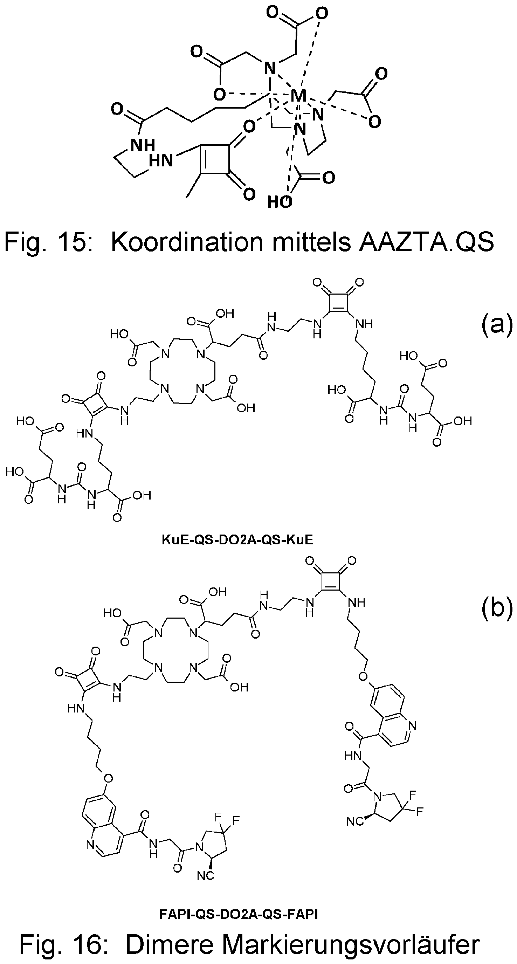

Chelatorzentrum vor unspezifischer Koordination schützen. Dieser Effekt konnte bei der Radiomarkierung von TRAP.QS bei unterschiedlichen Temperaturen beobachtet werden. TRAP komlexiert bei Raumtemperatur quantitativ. Demgegenüber wurde unter gleichen Bedingungen bei TRAP.QS ein RCY-Wert von lediglich 50% gemessen. Wird die Temperatur erhöht, so steigt die Markierungsausbeute von TRAP.QS auf quantitative Werte an. Hieran zeigt sich der Einfluss, den die Quadratsäure auf die Komplexierung hat. Dieser in Fig. 15 illustrierte Effekt ermöglicht die stabile Komplexierung von Metallen mit hoher Koordinationszahl, wie beispielsweise Zirkonium mithilfe des Chelators AAZTA.QS (Fig. 15: Koordination mittels AAZTA.QS).

### Beispiel 7: Dimere Markierungsvorläufer mit jeweils zwei KuE- und FAPI-Targetingvektoren

### (Fig. 16a: Dimerer Markierungsvorläufer KuE-QS-DOA2A-QS-KuE; Fig. 16b: Dimerer Markierungsvorläufer FAPI-QS-DOA2A-QS-FAPI)

(i) Synthese der DO2A-Einheit mit zwei Amin-Seitengruppen:

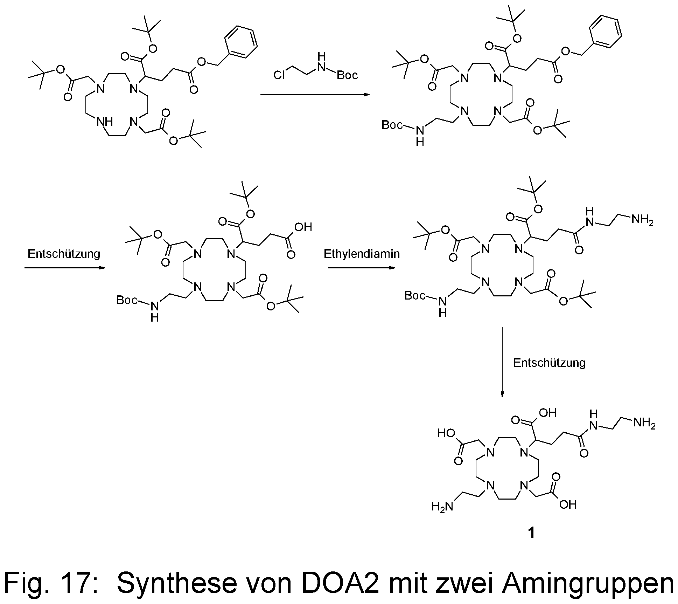

   (Fig. 17: Synthese von DOA2 mit zwei Amingruppen)
(ii) Synthese des KuE-QS-Motifs:

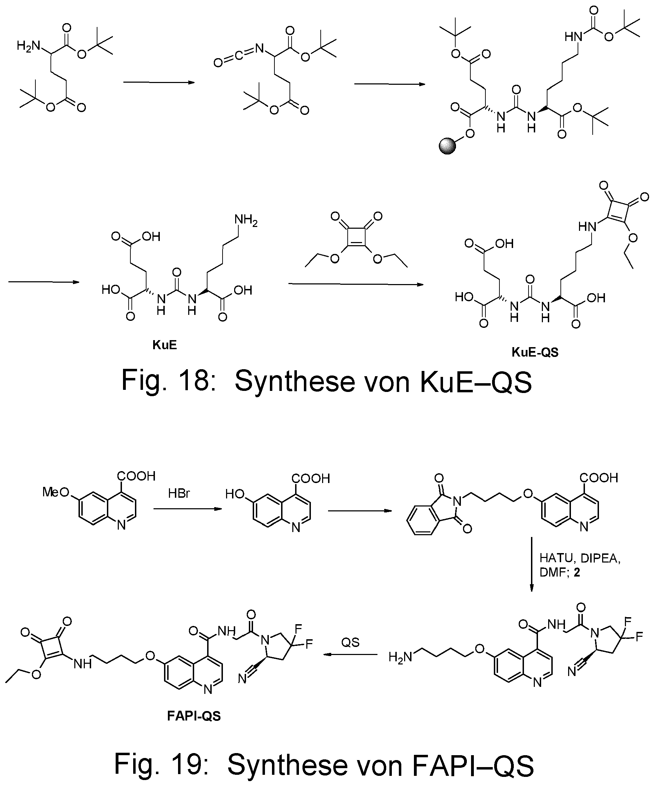

   (Fig. 18: Synthese von KuE-QS)
(iii) Synthese von FAPI-QS, Kopplung des 4,4-Difluoroprolin-quinolin-4-carbonsäure Motivs mit QS:
   (Fig. 19: Synthese von FAPI-QS)
(iv) Kopplung der DO2A-Einheit mit KuE-QS und respektive FAPI-QS:

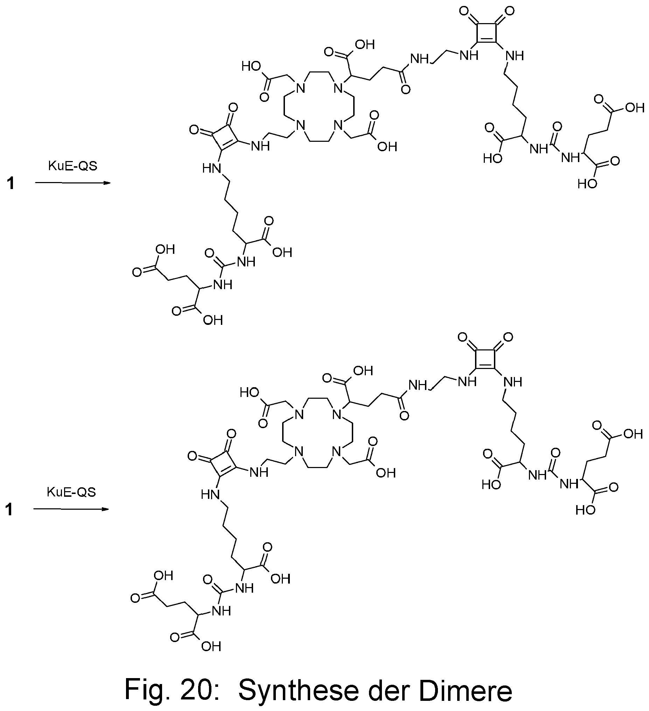

   (Fig. 20: Synthese der Dimere)

### Nicht anspruchsgemäßes Beispiel 8: ⁶⁸Ga-DOTA.QS.PSMA präklinische Untersuchung

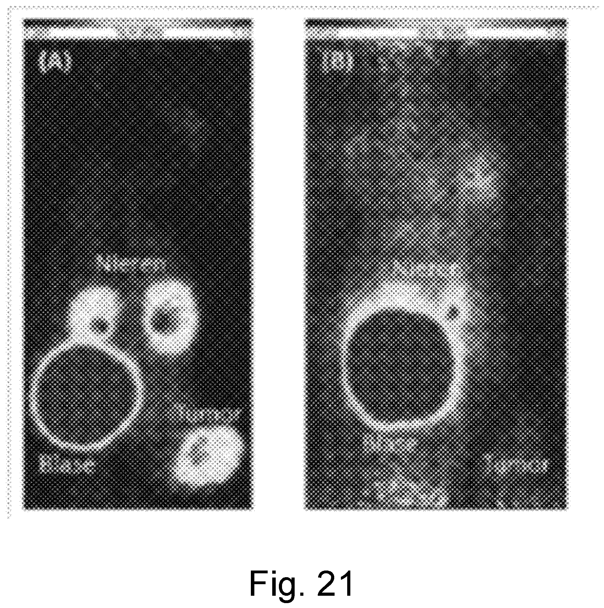

Mittels PET wurden präklinische Vergleichsversuche mit Radiotracern des Typs ⁶⁸Ga-DOTA.QS.PSMA, ⁶⁸Ga-PSMA-11 und ⁶⁸Ga-PSMA-617 an NMRInu/nu Nacktmäusen mit einem LNCaP-Tumor am rechten Hinterlauf durchgeführt. Fig. 21 zeigt PET-Aufnahmen 60 min nach Injektion des Tracers ⁶⁸Ga-DOTA.QS.PSMA, wobei Teilbild (A) und (B) die PET-Aufnahmen einer ungeblockten und respektive einer mittels koinjiziertem 2-PMPA geblockten Tumormaus wiedergeben.

**Tabelle 1: Standardisierte Aufnahmewerte (SUV) von PSMA-Tracern**

| | **SUV** | | |
|---|---|---|---|
| | **DOTA.QS.PSMA** | **PSMA-11** | **PSMA-617** |
| **Tumor** | 0,73 | 1,16 | 0,73 |
| **Nieren** | 0,43 | 4,71 | 0,27 |
| **Leber** | 0,27 | 0,25 | 0,29 |

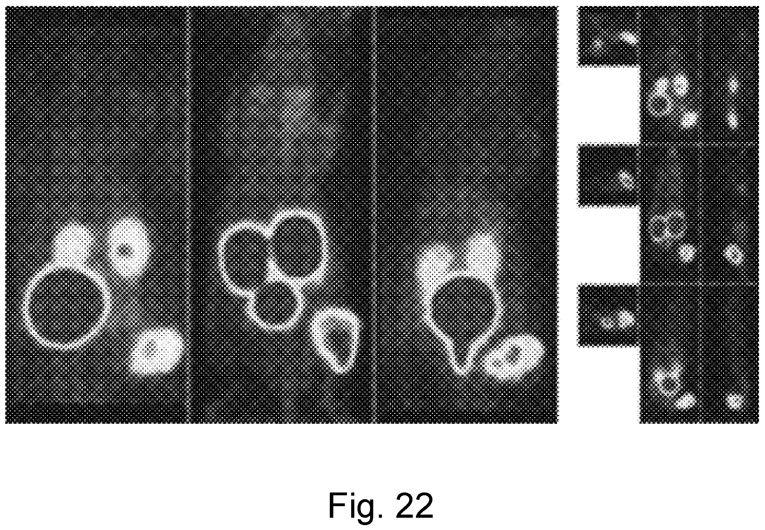

Aus den in Fig. 22 wiedergegebenen PET-Aufnahmen ist ersichtlich, dass sich der Tracer im Tumor stark anreichert. Aus den PET-Daten wurde für ⁶⁸Ga-DOTA.QS.PSMA ein standardisierter Aufnahmewert (standardized uptake value, SUV) von 0,73 im Tumor ermittelt. Durch Extraktion der Organe und Messung von Gewicht und Aktivität bestimmte biologische Verteilungsdaten zeigen für ⁶⁸Ga-DOTA.QS.PSMA eine geringfügig niedrigere bzw. gleiche Tumoraktivität wie ⁶⁸Ga-PSMA-11 und ⁶⁸Ga-PSMA-617. Demgegenüber ist die Off-Target-Aktivität in der Niere deutlich geringer als bei ⁶⁸Ga-PSMA-11.

Im Vergleich zu anderen bekannten Radiotracern ist die Off-Target-Anreicherung von ⁶⁸Ga-DOTA.QS.PSMA in Niere und Leber deutlich reduziert. ⁶⁸Ga-DOTA.QS.PSMA weist eine hohe Affinität zu Tumorgewebe auf und verbessert den Kontrast bzw. das Signal-RauschVerhältnis in der bildgebenden PET-Diagnostik von PCa-Primärtumoren und insbesondere von PCa-befallenen Lymphknoten im Beckenbereich. Auch wird die Strahlenbelastung der Niere und benachbarter Organe gemindert, was für die theranostische Behandlung einen erheblichen Vorteil darstellt.

Analoge Untersuchungen mit ⁶⁴Cu-TRAP.QS.PSMA und ⁶⁸Ga-NOTAGA.QS.PSMA lieferten vergleichbare Ergebnisse. Im Weiteren wurde DOTA.QS.PSMA mit ¹⁷⁷Lu und ²²⁵Ac markiert. Erste Resultate zur radiologischen und physiologischen Stabilität dieser Tracer indizieren deren Eignung für die Theranostik.

Aufgrund des Einflusses der aromatischen Bindungstasche von PSMA auf die Affinität von PSMA-Inhibitoren wird der Lipophilie von PSMA-Tracern einige Bedeutung beigemessen. Studien deuten daraufhin, dass eine erhöhte Lipophilie zudem die Aufnahme bzw. Endozytose des Tracers in Tumorzellen begünstigt. Dementsprechend wurde die Lipophilie der Tracer TRAP.QS.PSMA und DOTA.QS.PSMA mittels der HPLC-Methode von Donovan und Pescatore (S. F. Donovan, M. C. Pescatore, J. Chromatogr. A 2002, 952, 47-61) bestimmt. Hierzu wurden die Retentionszeit von TRAP.QS.PSMA, DOTA.QS.PSMA sowie einiger Kalibrierstandards mit bekannter Lipophilie in einer ODP-HPLC-Säule mit einem Methanol/Wasser-Gradienten bei einem pH-Wert von 7 gemessen. Die durch lineare Regression der Retentionszeiten ermittelten logD-Werte für TRAP.QS.PSMA und DOTA.QS.PSMA sind in Tabelle 2 zusammen mit Literaturwerten für PSMA-11 und PSMA-617 wiedergegeben.

Da DOTA.QS.PSMA auf der ODP-HPLC-Säule keine Retention aufweist, ist für logD lediglich ein maximaler Wert angegeben. TRAP.QS.PSMA, PSMA-11 und PSMA-617 haben vergleichbare Lipophilie. Überraschenderweise ist die Aufnahme von TRAP.QS.PSMA in der Niere gegenüber PSMA-11 und PSMA-617 deutlich reduziert. Diese Beobachtung ist durch die geringfügigen Unterschiede der jeweiligen logD-Werte nicht erklärbar. Offenbar beinflusst nicht nur die Lipophilie die Affinität und Endozytose, sondern andere Wechselwirkungen wie π-π-Stapelung in der enzymatischen Bindungstasche spielen ebenfalls eine Rolle. Hierbei erscheint Quadratsäure wegen ihrer im Vergleich zu Phenyl geringen Größe vorteilhaft. Demgegenüber zeigt DOTA.QS.PSMA eine beträchtlich höhere Lipophilie in Verbindung mit einer mit PSMA-617 vergleichbaren Aufnahme in der Niere.

**Tabelle 2: Lipophilie von PSMA-Tracern**

| **Tracer** | **logD [nM]** |
|---|---|
| TRAP.QS.PSMA | -1,5 ± 0,5 |
| DOTA.QS.PSMA | ≤ -3,5 |
| PSMA-11 | -1,7 ± 0,6 |
| PSMA-617 | -2,0 ± 0,3 |

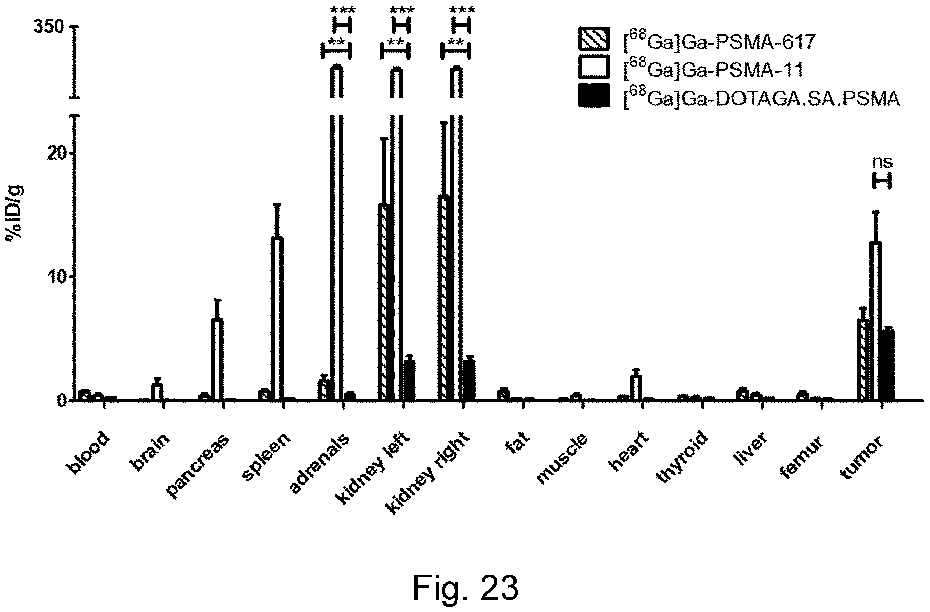

Zudem wurden mittels PET wurde präklinische ex vivo Versuche mit den Radiotracer des Typs [⁶⁸Ga]Ga-DOTA.QS.PSMA, [⁶⁸Ga]Ga-PSMA-11 und [⁶⁸Ga]Ga-PSMA-617 an NMRInu/nu Nacktmäusen mit eine LNCap Tumor durchgeführt. Fig. 23 zeigt die Organverteilungen der entsprechenden Verbindungen. Die erhaltenen Ergebnisse unterstreichen die bei den in vivo Versuchen erhaltenen. Durch Extraktion der Organe und Messung von Gewicht und Aktivität bestimmte biologische Verteilungsdaten zeigen für [⁶⁸Ga]Ga-DOTA.QS.PSMA eine geringfügig niedrigere bzw. gleiche Tumoraktivität wie [⁶⁸Ga]Ga-PSMA-11 und [⁶⁸Ga]Ga-PSMA-617. Demgegenüber ist die Off-Target-Aktivität in der Niere deutlich geringer als bei [⁶⁸Ga]Ga-PSMA-11.

### Nicht anspruchsgemäßes Beispiel 9: FPPS-Tracer

Bisphosphonate, wie Alendronat, Pamidronat und Zoledronat (Strukturformel [39], [40] und respektive [41]) inhibieren Farnesyl-Pyrophosphat-Synthase (FPPS) und induzieren in Knochenmetastasen Apoptose.

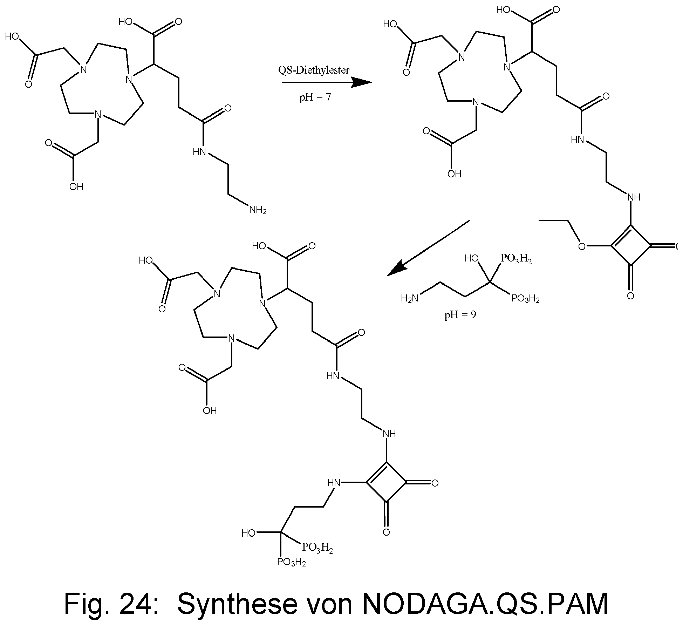

Für die Markierung von Knochenmetastasen wurde nach der in Beispiel 1 beschriebenen Synthesestrategie ein Quadratsäure-gekoppelter Tracer NOTA.QS.PAM mit Chelator NOTA und Targetingvektor Pamindronat (Strukturformel [40]) synthetisiert. Fig. 24 illustriert das angewendete Syntheseschema anhand des Chelators NODAGA (Fig. 24: Synthese von NODAGA.QS.PAM).

Der Tracer NOTA.QS.PAM und der in der klinischen Anwendung etablierte Referenztracer DOTA^{Zol} wurden mit ⁶⁸Ga markiert, jungen gesunden Wistar-Ratten injiziert und PET-Scans aufgezeichnet, jeweils in Zeitabständen von 5 min, 60 min und 120 min nach Injektion.

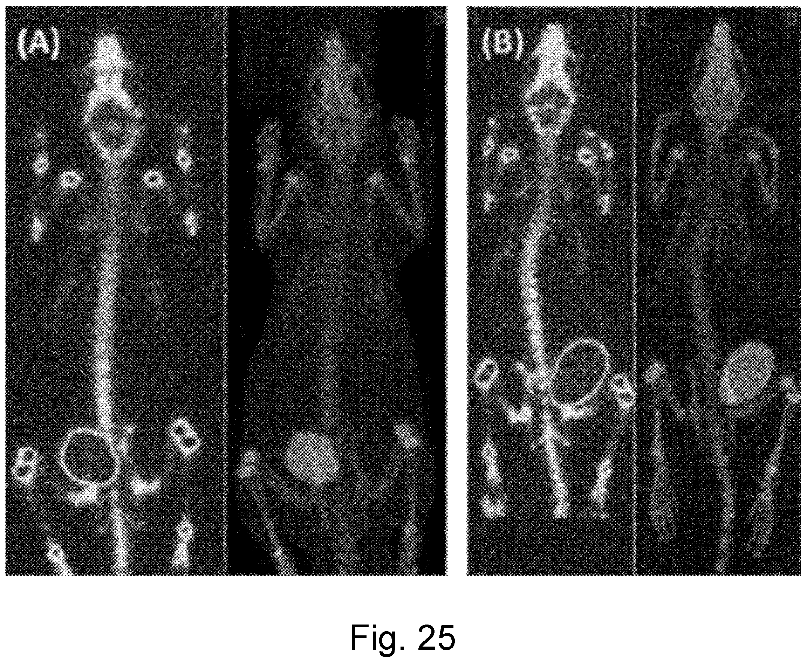

Fig. 25 zeigt die entsprechenden PET-Aufnahmen für die Tracer ⁶⁸Ga-NOTA.QS.PAM und ⁶⁸Ga-DOTA^{Zo1} 120 min nach Injektion. Beide Tracer zeigen eine spezifische Aufnahme in Knochenbereichen mit erhöhter Remodellierungsrate, insbesondere in den Epiphysen, die sich bei jungen Ratten noch im Wachstum befinden. Neben dem Skelett weist die Blase erhöhte Aktivität auf und indiziert die präferentiell renale Ausscheidung. Demgegenüber ist die Retention in Weichgewebe äußerst gering.

Im Vergleich zu ⁶⁸Ga-DOTA^{Zo1} ist die renale Ausscheidung von ⁶⁸Ga-NOTA.QS.PAM geringfügig reduziert. Diese Beobachtung steht in Einklang mit der renalen Ausscheidung von PSMA-Tracern. Diese ist das Ergebnis einer erhöhten Anreicherung im Zielgewebe in Verbindung mit beschleunigter renaler Ausscheidung von freiem, nicht spezifisch gebundenem Tracer. Hinsichtlich der pharmakologischen Kinetik weisen die Quadratsäure-gekoppleten Tracer Vorteile gegenüber bekannten Tracern auf.

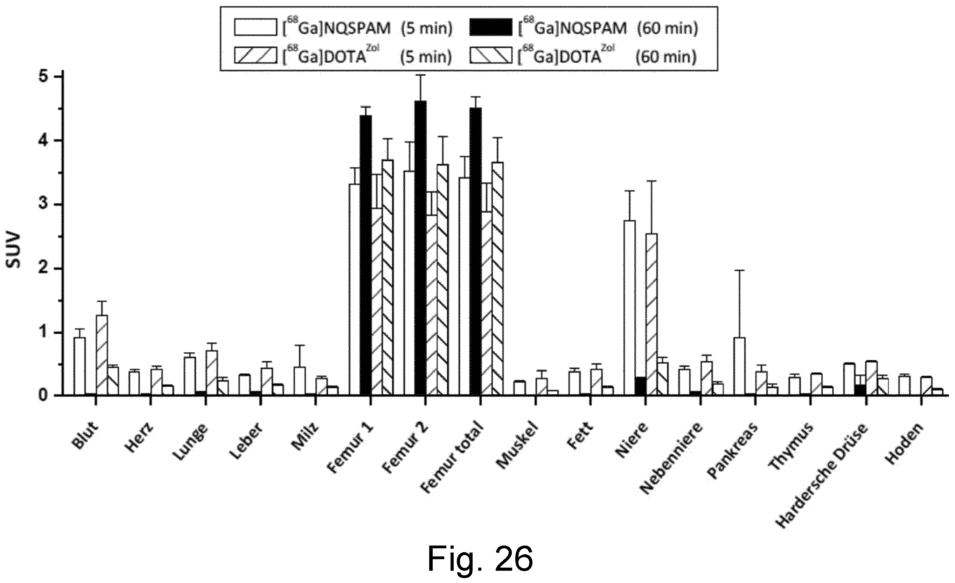

In Fig. 26 sind die Aufnahmewerte (SUV) für ⁶⁸Ga-NOTA.QS.PAM und ⁶⁸Ga-DOTA^{Zo1} in verschiedenen Organen in Zeitabständen von 5 min und 60 min nach Injektion in Form eines Balkendiagramms wiedergegeben. Die Organverteilung der Aktivität zeigt, dass beide Tracer eine hohe Aufnahme und Retention in Knochen aufweisen, wobei ⁶⁸Ga- NOTA.QS.PAM einen geringfügig höheren Femur-SUV hat (SUV Femur: ⁶⁸Ga-DOTA^{Zo1} = 3,7 ± 0,4; ⁶⁸Ga-NOTA. QS.PAM = 4,5 ± 0,2). Beide Tracer zeichnen sich durch renale Ausscheidung und geringe Retention im restlichen Gewebe aus.

### Nicht anspruchsgemäßes Beispiel 10: QS.PSMA

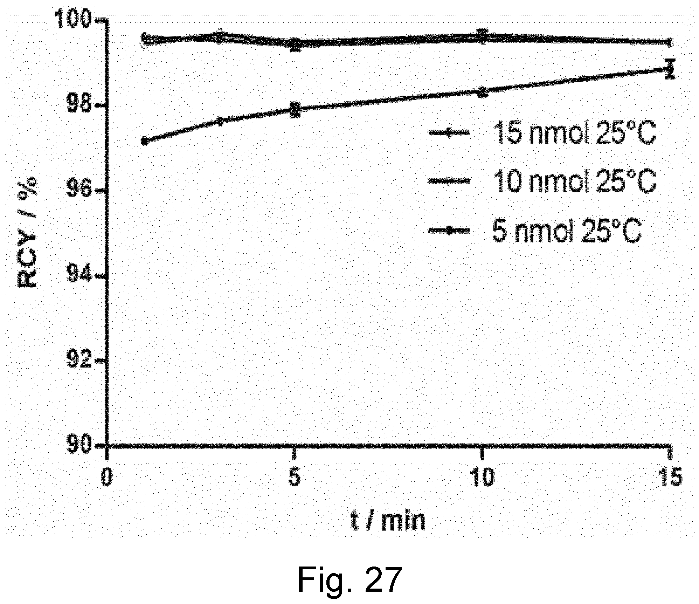

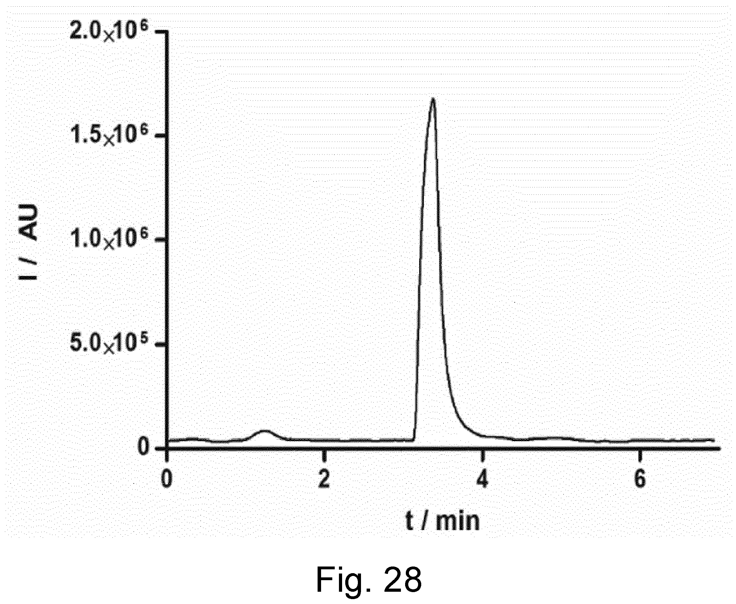

Um die Aktivität der QS zu verdeutlichen wurden vergleichbare Versuche wie bei DOTA.QS.PSMA mit NODAGA.QS.PSMA durchgeführt. Fig. 27 und 28 zeigen die radioaktive Markierung der Verbindungen mit ⁶⁸Ga jeweils gemessen mit radio-DC (Fig. 27) und radio-HPLC (Fig. 28). Es werden Ausbeute von mehr als 95% erreicht.

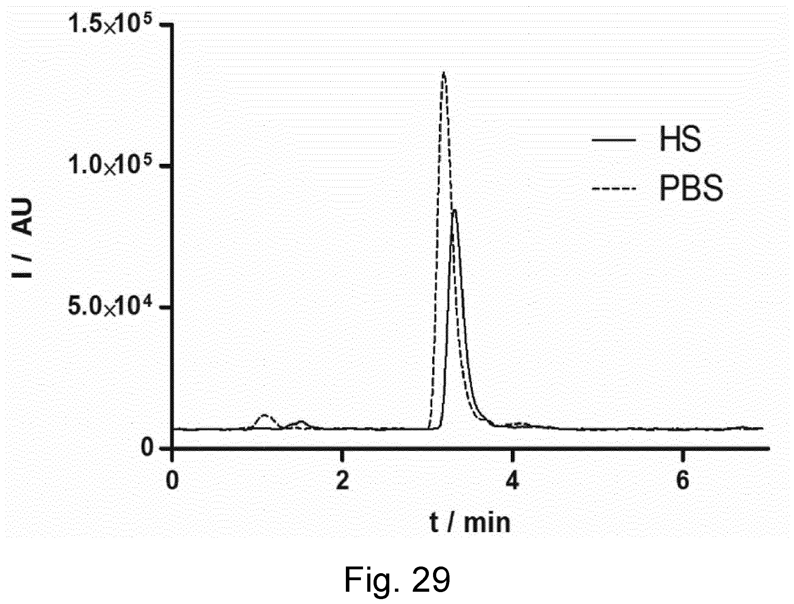

Entsprechende Stabilitätstests wurde durchgeführt in humanem Serum und in PBS-Puffer. Die Verbindungen zeigen Stabilitäten von mehr als 95% nach 2h in PBS und in HS. Fig. 29 zeigt die mittels HPLC gemessene Stabilität.

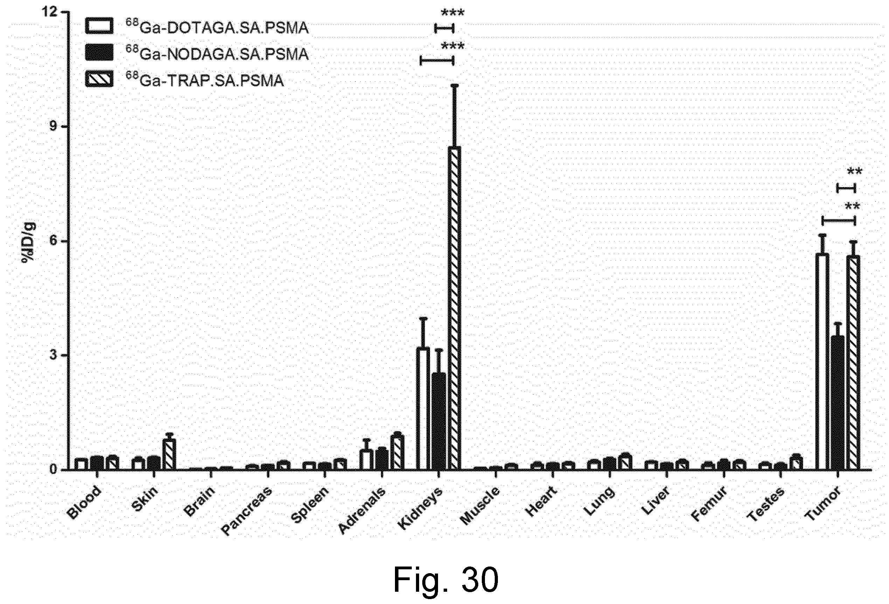

Im Weiteren wurden die drei Verbindungen DOTAGA.QS.PSMA, NODAGA.QS.PSMA und TRAP.QS.PSMA in vivo und ex vivo untersucht. Fig. 30 zeigt die ex vivo Resultate der drei jeweils mit ⁶⁸Ga markierten Verbindungen. Aus Fig. 30 ist ersichtlich, dass alle drei Verbindungen sich im Tumorgewebe anreichern, wobei NODAGA.QS.PSMA eine niedrige Anreicherung in den Nieren zeigt.

### Nicht anspruchsgemäßes Beispiel 11: TRAP.QS.PSMA

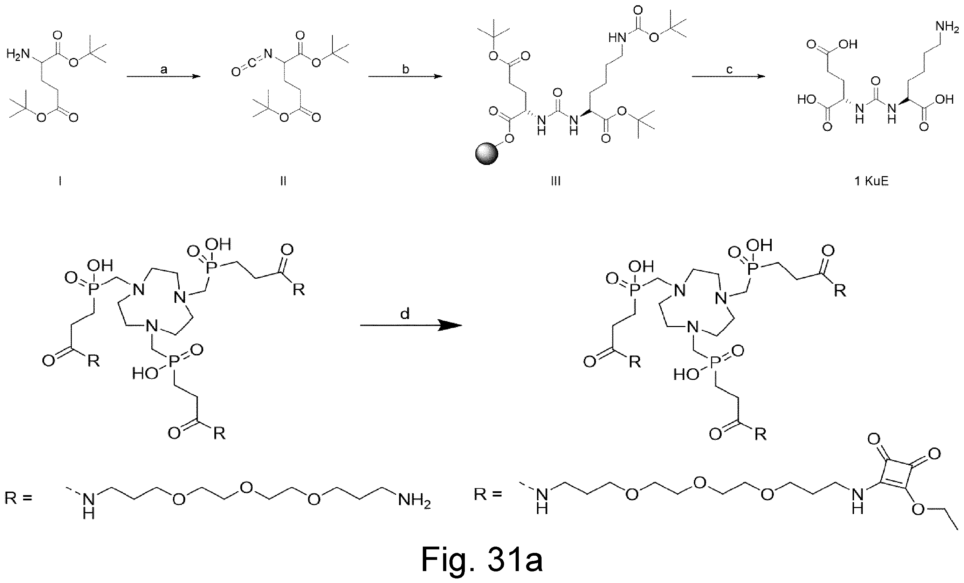

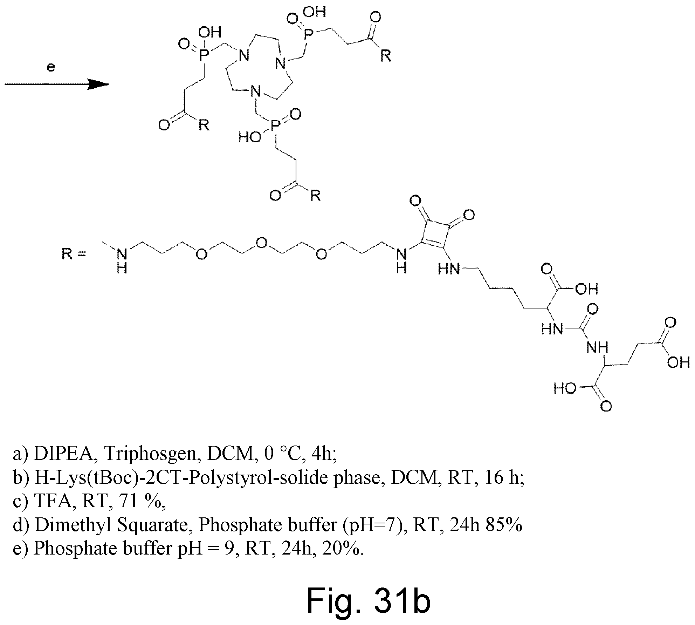

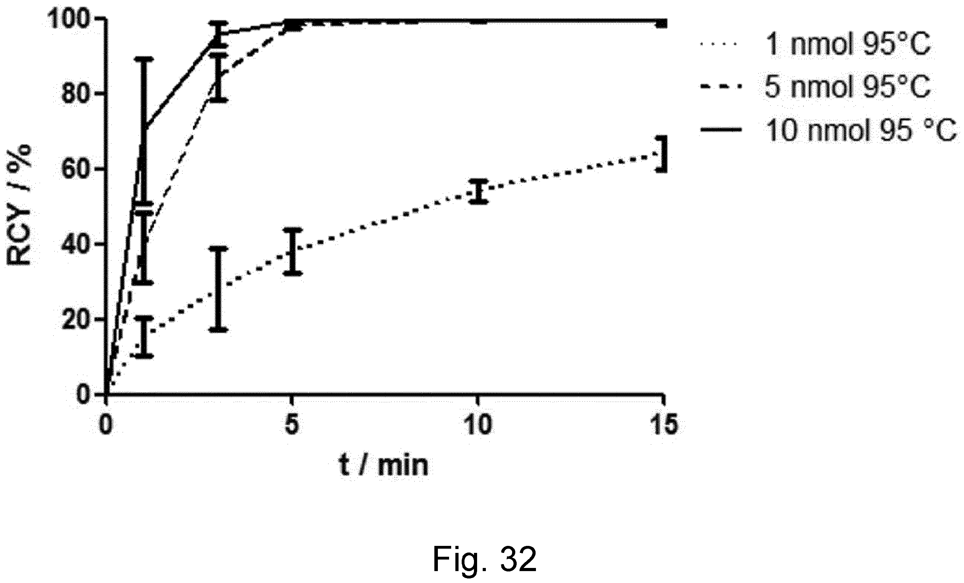

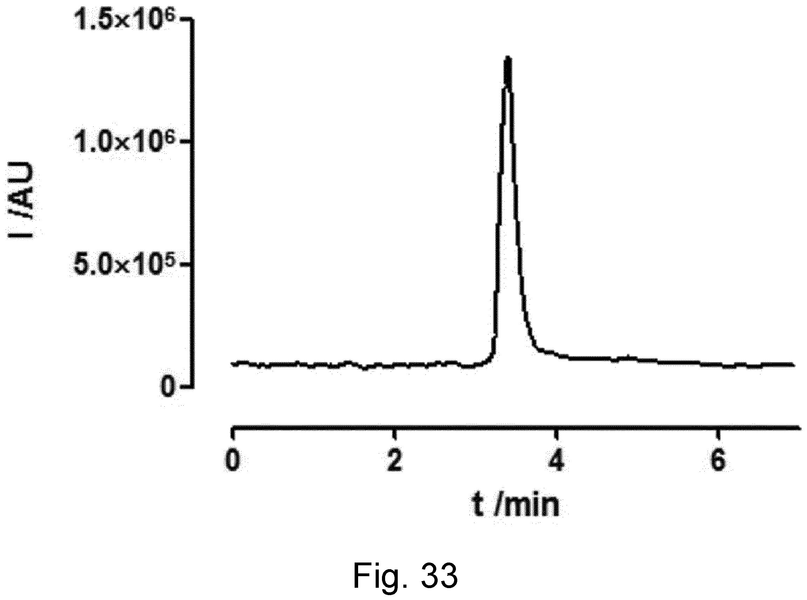

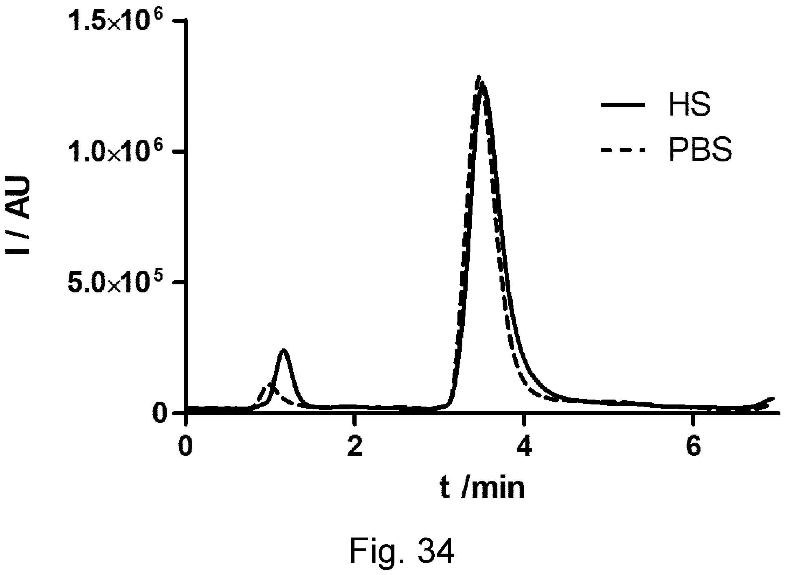

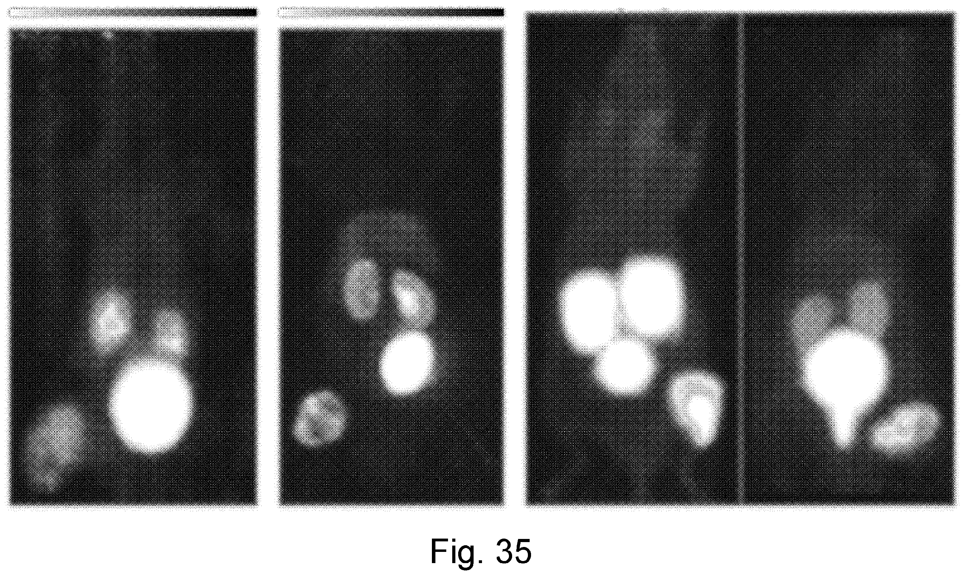

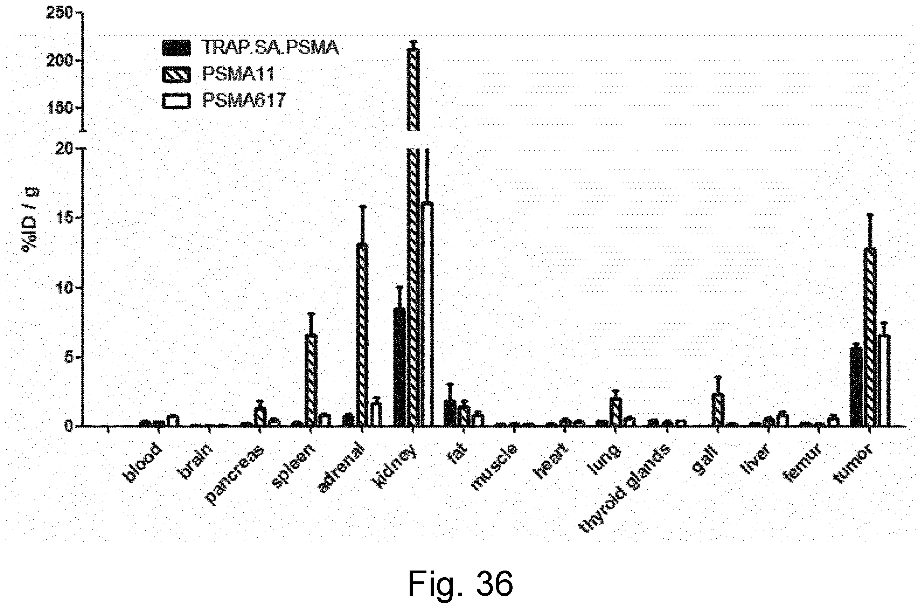

Fig. 31a-b, 32-36 zeigen die Synthese sowie Messergebnisse zur Radiomarkierung, Stabilität und in vivo Untersuchungen des Radiotracer [⁶⁸Ga]Ga-TRAP.QS.PSMA. Die Ergebnisse sind vergleichbar mit denen der Beispiele 8 und 10. Die in vivo Untersuchungen wurden mit ⁶⁸Ga und ⁶⁴Cu durchgeführt. Aus den Aufnahmen ist ersichtlich, dass im Vergleich zu den Beispielen 8 und 10 die Anreicherung in den Nieren für beide Radiotracer erhöht ist. Dies ist auf die abweichende, durch langkettige Linker verursachte Lipohilie der Radiotracer des Beispiels 11 zurückzuführen. Der ex vivo Vergleich zeigt, dass gegenüber [⁶⁸Ga]Ga-PSMA-11 die Anreicherung im Tumorgewebe erhöht und in den Nieren merklich erniedrigt ist.

### Nicht anspruchsgemäßes Beispiel 12: [⁶⁸ Ga]Ga-DATA.QS.PSMA

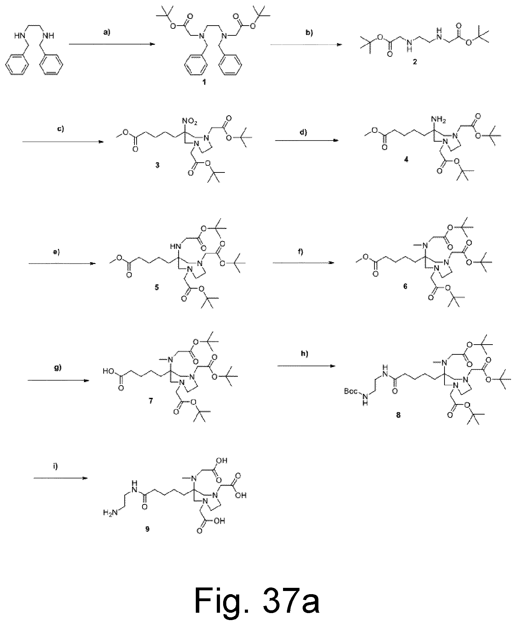

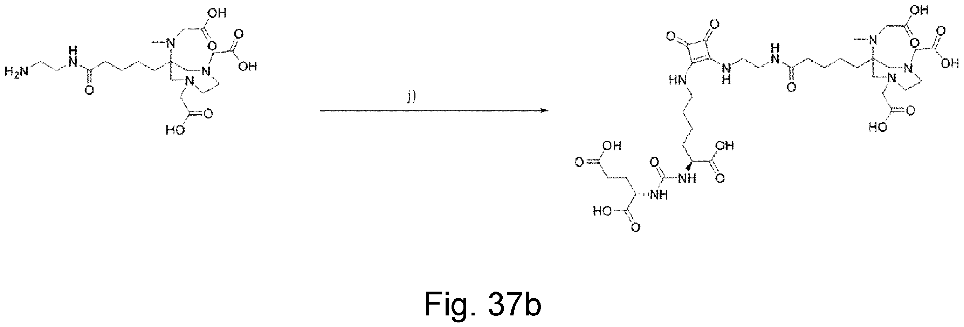

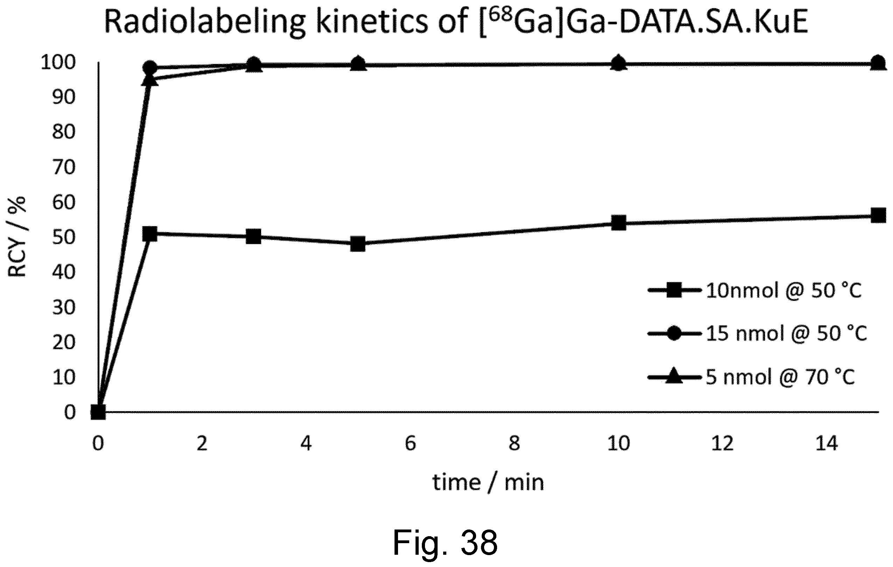

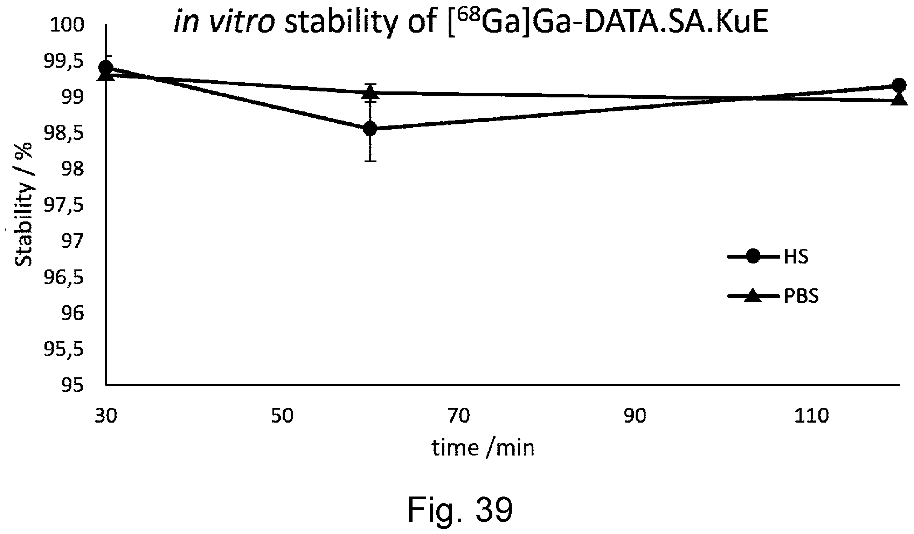

Weitere Verbindungen sind jene des Typs DATA.QS.PSMA, deren Struktur den anderen aufgeführten Verbindungen entspricht, wobei der Chelator DATA eine einfachere und mildere Markierung ermöglicht. Bei der in Fig. 37 dargestellten Synthese wird eine Ausbeute von etwa 70% erzielt. Bei der radioaktiven Markierung mit ⁶⁸Ga wird eine Ausbeute von mehr als 95% erreicht (Fig. 38). Wie aus Fig. 39 ersichtlich, weisen die Verbindungen eine hohe Stabilität sowohl in Humanserum (HS) wie auch phosphatgepufferter Salzlösung (PBS) auf. Bei vitro Untersuchungen der Verbindung mit LNCap-Zellen wird ein IC₅₀-Wert von 51.5 nM erhalten, der mit PSMA-11 und PSMA-617 vergleichbar ist (Tabelle 3).

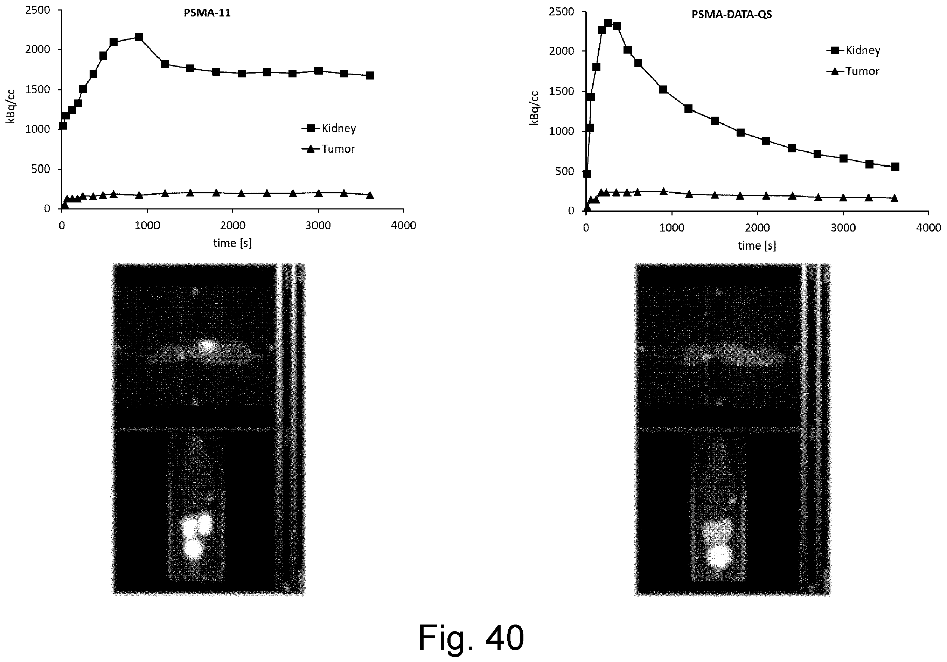

Desweiteren wurden Verbindungen des Typs DATA.QS.PSMA in vivo im gleichen Tiermodell mit PSMA-11 verglichen. Die MIP-Abbildungen (Fig. 40) zeigen deutlich, dass auch für [⁶⁸Ga]Ga-DATA.QS.PSMA eine sehr gute Anreicherung in den Tumor zu sehen ist. Aus den in Fig 40 wiedergegebenen Zeit/Aktivitätskurven ist zudem ersichtlich, dass die Ausscheidung über die Nieren für [⁶⁸Ga]Ga-DATA.QS.PSMA in der ersten Stunde deutlich schneller erfolgt als bei [⁶⁸Ga]-PSMA-11. Die Anreicherung im Tumorgewebe ist vergleichbar.

**Tabelle 3: IC₅₀-Werte der nicht markierten Verbindungen.**

| **Verbindung** | **IC₅₀ [nM]** |
|---|---|
| PSMA-11 | 26.1 ± 1.2 |
| PSMA-617 | 15.1 |
| DATA.QS.PSMA | 51.1±5.5 |

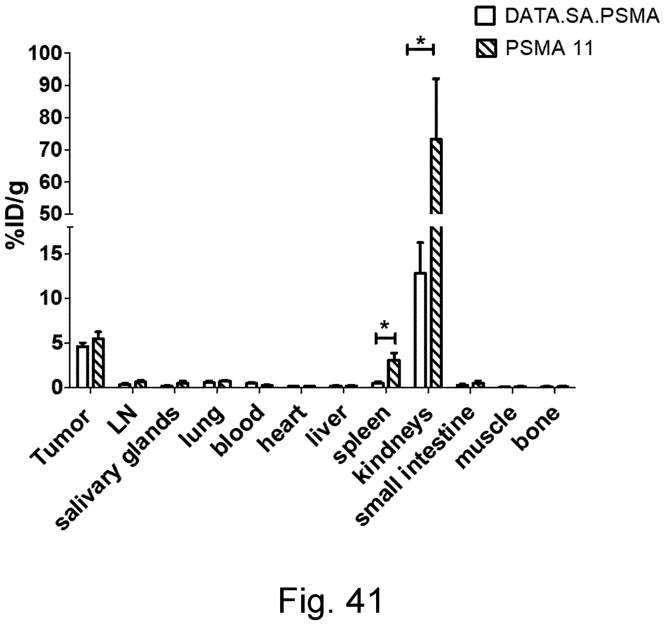

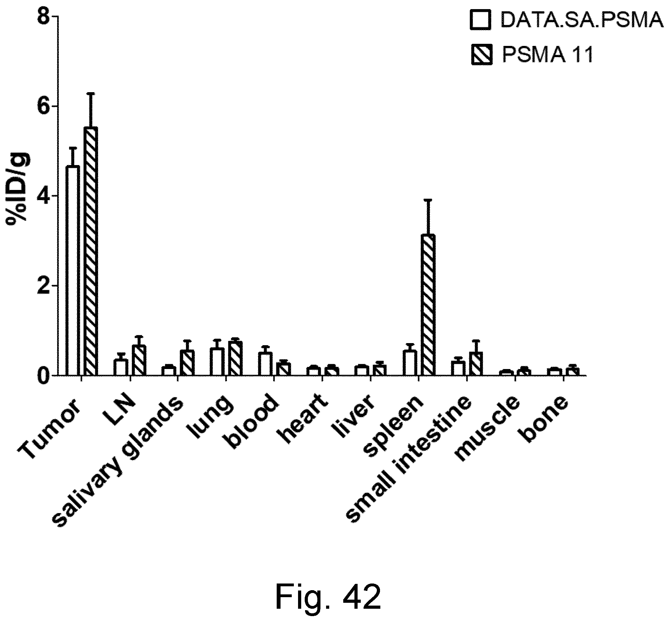

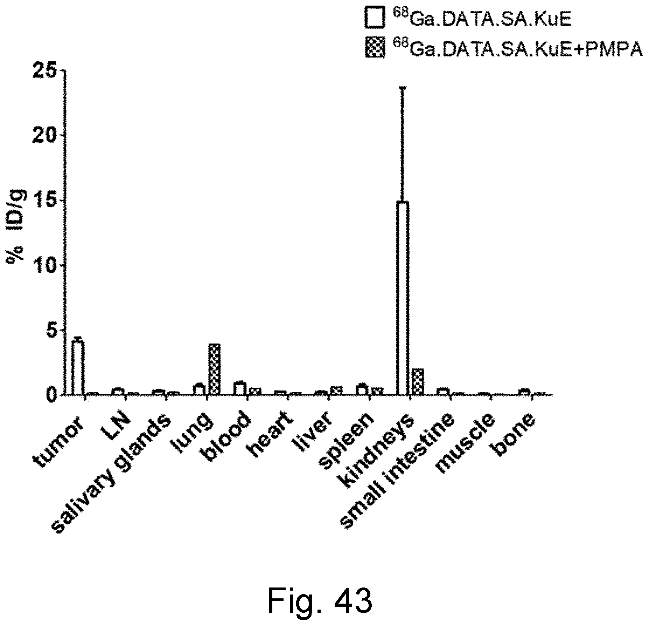

Die Ergebnisse von ex vivo Untersuchungen (Fig. 41-43) stehen in Einklang mit den in vivo Beobachtungen. Wie aus Tabelle 4 ersichtlich, sind die %ID/g-Werte im Tumor der beiden mit ⁶⁸Ga markierten Verbindungen vergleichbar, wohingegen bei [⁶⁸Ga]Ga-DATA.QS.PSMA die Anreicherung in den Nieren und den Speicheldrüsen erheblich reduziert ist. Niedrige Anreicherung in den Speicheldrüsen ist sehr vorteilhaft, da letztere bei bekannten Verfahren zur radiopharmazeutischen Behandlung von Prostatakrebs einer erhöhten Dosis ausgesetzt und in ihrer Funktion erheblich beeinträchtigt sind. Blocking-Studien mit 2-PMPA zeigen zudem, dass die Verbindungen eine erhöhte Spezifizität für PSMA aufweisen.

**Tabelle 4: Ex vivo Aktivitäten**

| | **⁶⁸Ga.DATA.QS.PSMA** | **⁶⁸Ga.PSMA 11** |
|---|---|---|
| | **% ID/g ± SD** | |
| **tumor** | 4.65 ± 0.58 | 5.51 ± 0.38 |
| **LN** | 0.35 ± 0.20 | 0.66 ± 0.10 |
| **salivary glands** | 0.19 ± 0.07 | 0.54 ± 0.11 |
| **lung** | 0.59 ± 0.27 | 0.74 ± 0.04 |
| **blood** | 0.50 ± 0.19 | 0.27 ± 0.03 |
| **heart** | 0.17 ± 0.06 | 0.17 ± 0.03 |
| **liver** | 0.21 ± 0.04 | 0.23 ± 0.04 |
| **spleen** | 0.54 ± 0.22 | 3.12 ± 0.39 |
| **kidney left** | 6.59 ± 2.45 | 36.66 ± 5.05 |
| **kidney right** | 6.23 ± 2.39 | 36.72 ± 4.33 |
| **small intestine** | 0.31 ± 0.12 | 0.50 ± 0.14 |
| **muscle** | 0.09 ± 0.05 | 0.11 ± 0.03 |
| **bone** | 0.15 ± 0.03 | 0.15 ± 0.04 |

### Nicht anspruchsgemäßes Beispiel 13: [⁴⁴Sc]Sc-AAZTA.QS.PSMA

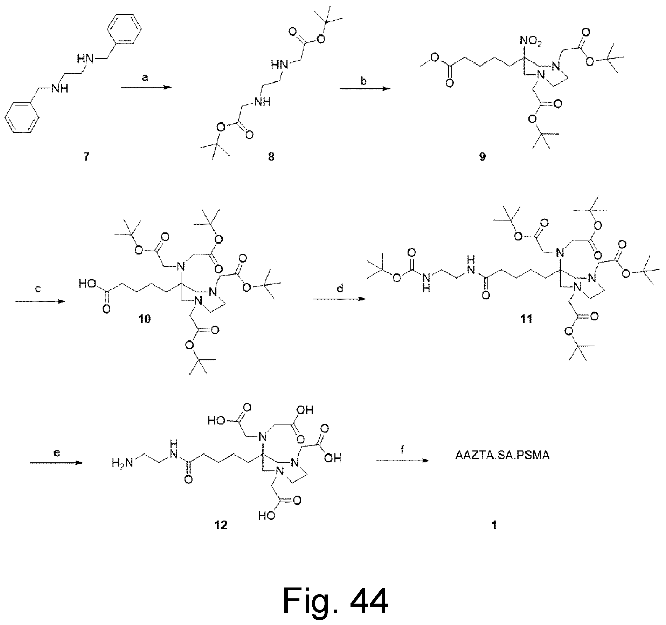

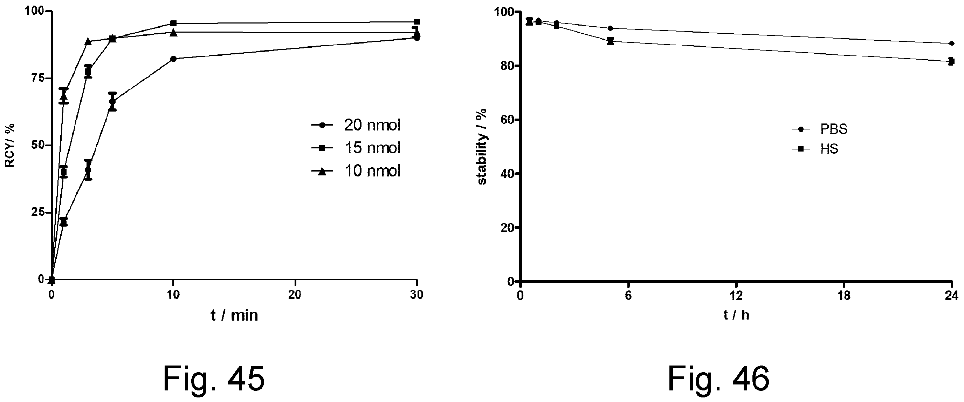

Ähnlich wie DATA lässt sich auch der Chelator AAZTA bei milden Bedingungen mit Radionukliden, wie beispielsweise ⁴⁴Sc und ⁶⁸Ga markieren. In diesem Beispiel wurde für die Markierung ⁴⁴Sc eingesetzt und die Eigenschaften des Radiotracers [⁴⁴Sc]Sc-AAZTA.QS.PSMA untersucht. Die in Fig. 44 dargestellte Synthese konnte einfach und mit hohen Ausbeuten durchgeführt werden. Wie in Fig. 45 gezeigt, wird auch bei der Radiomarkierung eine hohe Ausbeute erhalten. Die Stabilität von [⁴⁴Sc]Sc-AAZTA.QS.PSMA beträgt mehr als 95% über einen Zeitraum von 24h (Fig. 46).

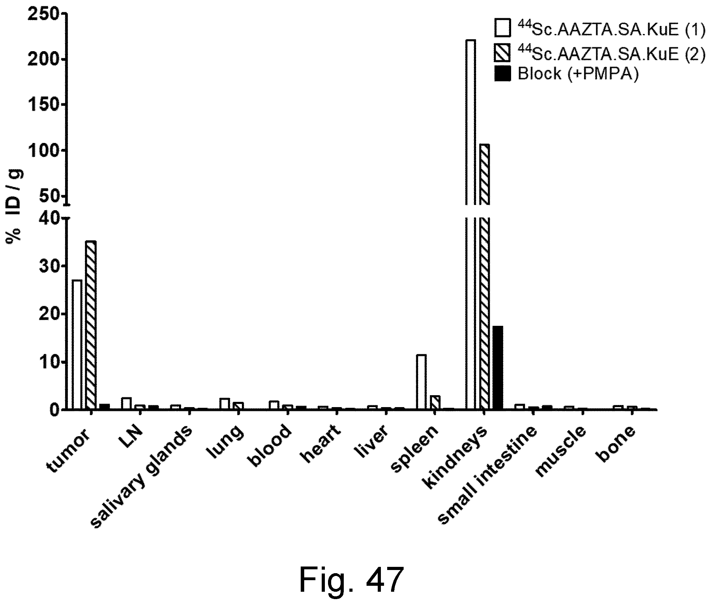

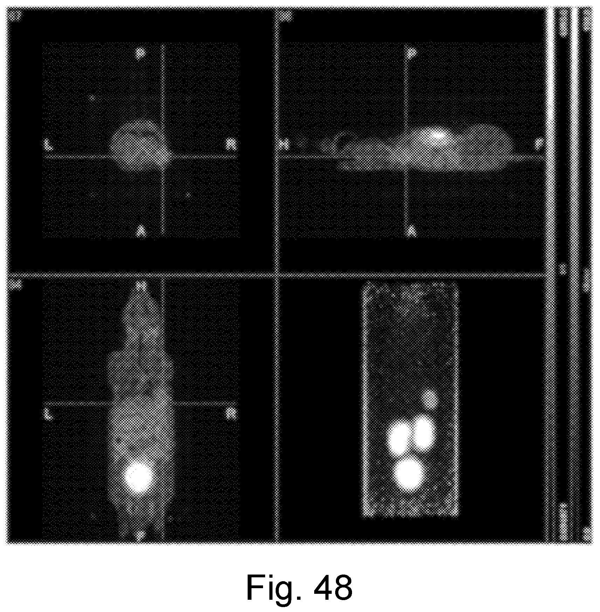

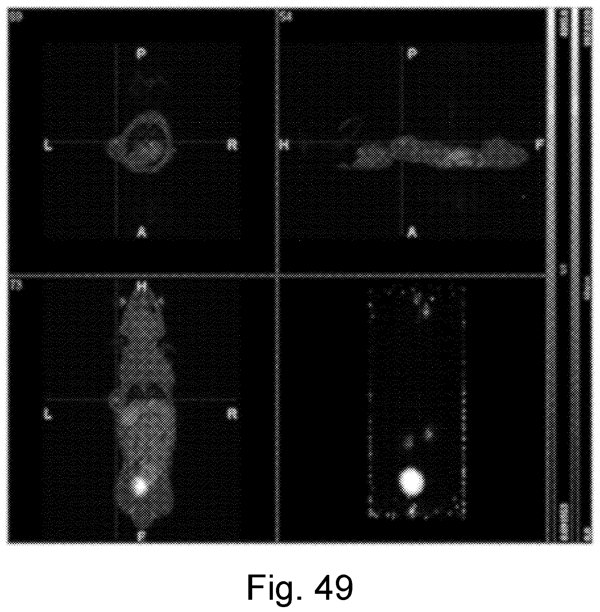

Der Radiotracer [⁴⁴Sc]Sc-AAZTA.QS.PSMA wurde außerdem in vivo in drei, jeweils einen LNCap Tumor tragenden Mäusen untersucht. An einer der Mäuse wurden zudem Blocking-Untersuchungen durchgeführt. Die in Tabelle 5 und Fig. 47 gezeigten ex vivo Ergebnisse zeigen, dass auch die mit ⁴⁴Sc markierten AAZTA-Derivate im Tumorgewebe stark angereichert werden. Außerdem kann mit 2-PMPA ein Großteil der Aktivität im Tumor geblockt werden. Gleiches gilt auch für die Nieren. Diese Resultate stehen in Einklang mit entsprechenden in vivo Untersuchungen. Fig. 48 und 49 zeigen die Tumoraktivität 1h nach Injektion ohne Blocking bzw. 40 nach Injektion (20 min statische Aufnahme) mit Blocking durch Koinjektion von 2-PMPA.

**Tabelle 5: Ex vivo Aktivitäten**

| | % ID/g | | |
|---|---|---|---|
| | ⁴⁴Sc.AAZTA.QS.KuE (1) | ⁴⁴Sc.AAZTA.QS.KuE (2) | Block (+PMPA) |
| tumor | 14,73 | 14,14 | 0,53 |
| LN | 1,33 | 0,38 | 0,39 |
| salivary glands | 0,51 | 0,18 | 0,11 |
| lung | 1,31 | 0,59 | 0,06 |
| blood | 0,96 | 0,41 | 0,34 |
| heart | 0,41 | 0,14 | 0,11 |
| liver | 0,42 | 0,16 | 0,16 |
| spleen | 6,19 | 1,15 | 0,15 |
| kindneys | 119,86 | 42,76 | 7,97 |
| small intestine | 0,62 | 0,22 | 0,35 |
| muscle | 0,38 | 0,13 | 0,05 |
| bone | 0,46 | 0,29 | 0,10 |

### Nicht anspruchsgemäßes Beispiel 14: Verbindungen für ¹⁸F-Markierung

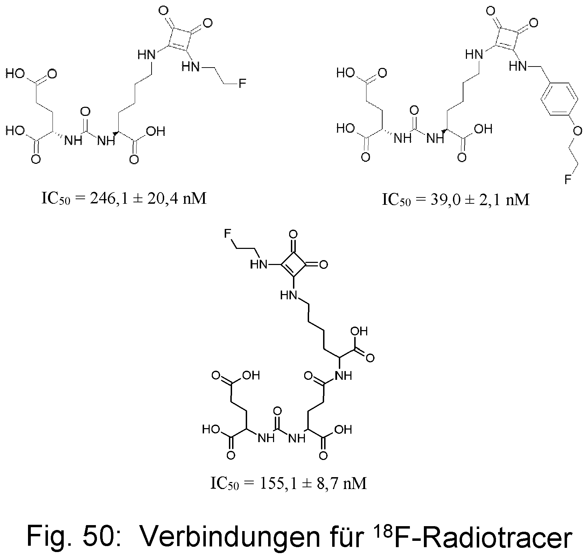

Für die PET-Diagnostik mit ¹⁸F wurden diverse Markierungsvorläufer synthetisiert und in vitro an LNCap Zellen untersucht. Hierbei wurden für mehrere der untersuchten Verbindungen niedrige, zu PSMA-11 und PSMA-617 korrespondierende IC₅₀-Werte beobachtet. Drei derartige Verbindungen mit ihren IC₅₀-Werten sind in Fig. 50 gezeigt (Fig. 50: Verbindungen für ¹⁸F -Radiotracer).

### Nicht anspruchsgemäßes Beispiel 15: DOTA.FAPi und DATA.FAPi

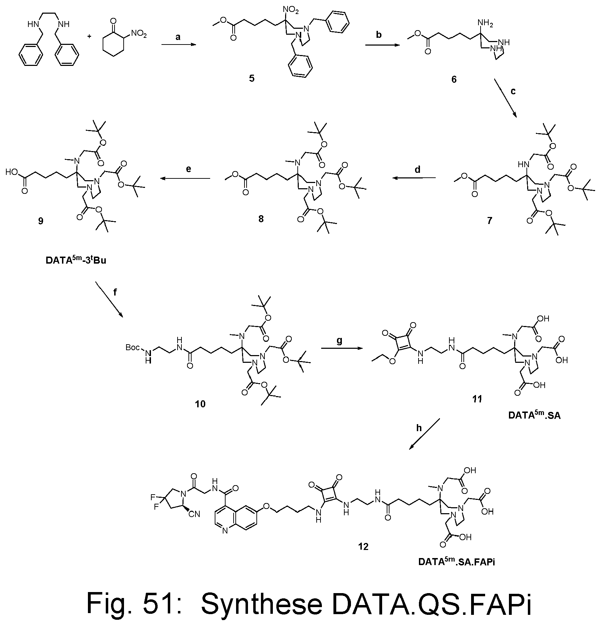

Die in Fig. 51 gezeigte Synthese von DOTA.QS.FAPi wird analog zu Beispiel 6 durchgeführt und umfasst die Schritte: (a) Paraformaldehyd, MeOH, Amberlyst A21; (b) Pd/C, CH3COOH, abs. EtOH, K2CO3; (c) tert-Butylbromoacetat, MeCN, K2CO3; (d) Formalin (37 wt%), CH3COOH, NaBH4, MeCN; (e) 1 M LiOH, 1,4-Dioxan/H2O (2:1); (f) N-boc-Ethylenediamin, HATU, HOBt, DIPEA, MeCN; (g) (i) 80 % TFA in DCM, (ii) 3,4-Diethoxycyclobut-3-ene-1,2-dion, Phosphat-Puffer pH 7, 1 M NaOH; (h) 3, Phosphate-Puffer pH 9, 1 M NaOH (Fig. 51: Synthese DATA.QS.FAPi).

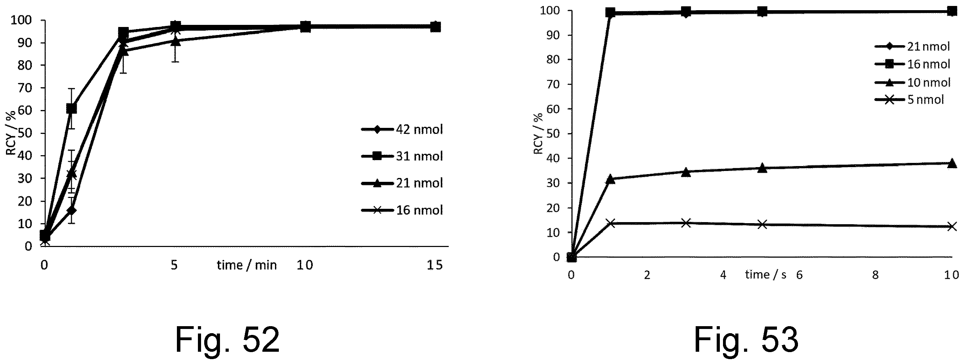

Die Markierung mit ⁶⁸Ga erfolgt schnell und mit hoher Ausbeute (Fig. 52 und 53). Die Stabilität in humanem Serum (HS) und NaCl-Lösung beträgt über einen Zeitraum von 2h mehr als 98% (Tabelle 6).

**Tabelle 6: Stabilität in HS, EtOH und NaCl**

| | **Medium** | | |
|---|---|---|---|
| **time/min** | **HS** | **EtOH** | **0.9 % NaCl** |
| 15 | 99.7 ± 0.3 | 99.6 ± 0.1 | 99.6 ± 0.2 |
| 30 | 99.8 ± 0.1 | 99.9 ± 0.1 | 99.9 ± 0.0 |
| 45 | 99.6 ± 0.4 | 99.9 ± 0.1 | 99.9 ± 0.1 |
| 60 | 99.2 ± 0.2 | 99.6 ± 0.2 | 99.6 ± 0.2 |
| 90 | 98.3 ± 0.2 | 99.6 ± 0.1 | 99.3 ± 0.1 |
| 120 | 98.8 ± 0.6 | 100.0 ± 0.1 | 99.4 ± 0.3 |

Die Messung der FAP IC₅₀-Werte wurde mit Z-Gly-Pro-7-amino-4-methylcoumarin (AMC) durchgeführt. Die Bestimmung der PREP IC₅₀-Werte erfolgte mit N-succinyl-Gly-Pro-AMC. Die Selektivitätsindices sind vergleichbar mit Literaturwerten (Jansen et al. J Med Chem, 2014, 7, 3053). Die Messwerte sind in Tabelle 7 wiedergegeben.

**Tabelle 7: IC50-Werte und Selektivitätsindices**

| | IC₅₀ FAP (nM)^{∗} | IC₅₀ PREP (µM) | Selektivitätsindex (FAP/PREP) |
|---|---|---|---|
| DOTA.QS.FAPi - uncomplexed | 0.9 ± 0.1 | 5.4 ± 0.3 | 6000 |
| DOTA.QS.FAPi - natGa | 1.4 ± 0.2 | 8.7 ± 0.9 | 6214 |
| DOTA.QS.FAPi - natLu | 0.8 ± 0.2 | 2.5 ± 0.4 | 3125 |
| DATA5m.QS.FAPi - uncomplexed | 0.8 ± 0.2 | 1.69 ± 0.09 | 2113 |
| DATA5m.QS.FAPi - natGa | 0.7 ± 0.1 | 4.7 ± 0.3 | 6714 |

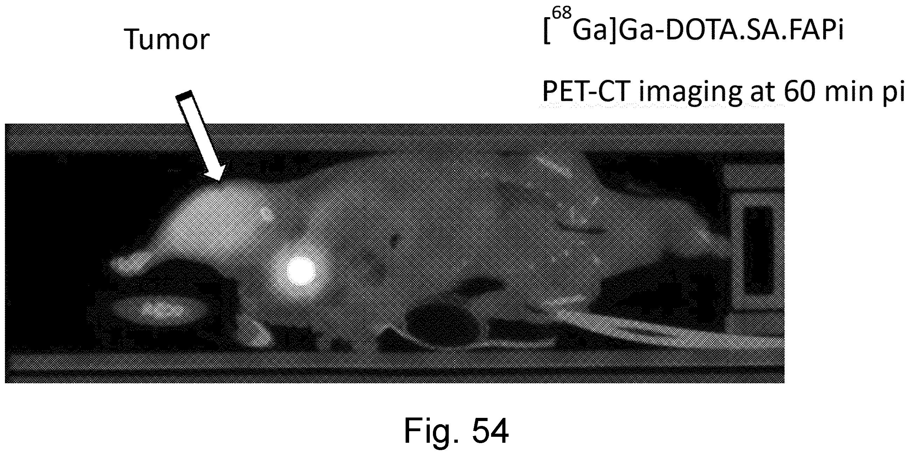

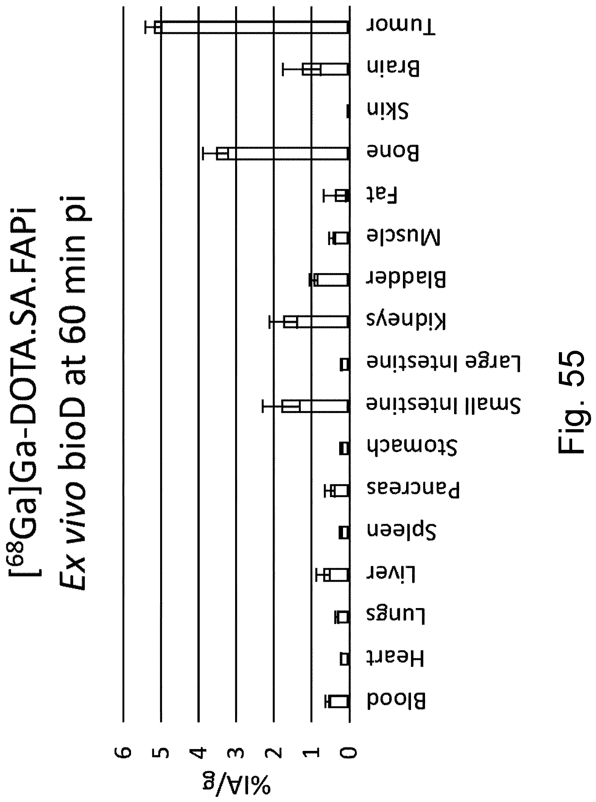

In vivo sowie ex vivo Untersuchungen mit [⁶⁸Ga]Ga-DOTA.QS.FAPi an Darmkrebs (HT29) tragenden Mäusen zeigen eine hohe Anreicherung im Tumorgewebe (Fig. 54 und 55).

## Patentansprüche

1. Markierungsvorläufer für ein Radiopharmakon mit der Struktur

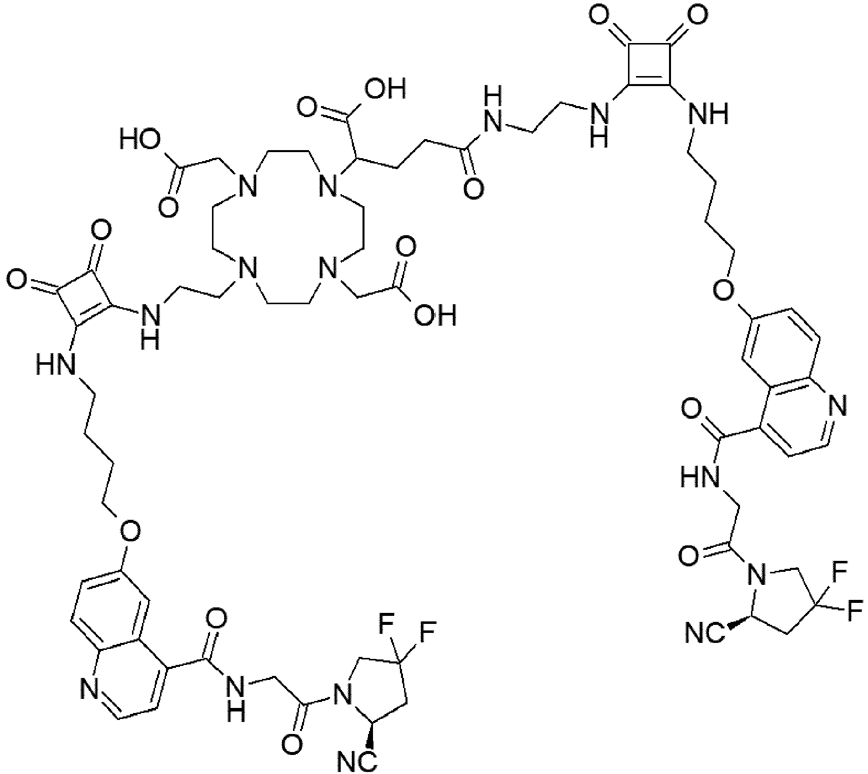

2. Radiotracer-Verbindung, umfassend den Markierungsvorläufer nach Anspruch 1 mit einem komplexierten Radioisotop, gewählt aus der Gruppe, umfassend ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy,¹⁶⁶Ho, ¹⁷⁵Yb, ¹⁷⁷Lu, ¹⁸⁶Re, ¹⁸⁸Re, ²¹³Bi und ²²⁵Ac.

3. Verwendung des Markierungsvorläufers nach Anspruch 1 zur Herstellung eines mit ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁴⁰Pr, ¹⁵⁹Gd, ¹⁴⁹Tb, ¹⁶⁰Tb, ¹⁶¹Tb, ¹⁶⁵Er, ¹⁶⁶Dy,¹⁶⁶Ho, ¹⁷⁵Yb, ¹⁷⁷Lu, ¹⁸⁶Re, ¹⁸⁸Re, ²¹³Bi oder ²²⁵Ac komplexierten Radiopharmakons.
